# EUROPEAN PATENT APPLICATION

(11) **EP 3 766 512 A1**
(43) Date of publication of application: **20.01.2021**
(21) Application number: 19186619.3
(22) Date of filing: 16.07.2019
(51) Int. Cl.: A61K 38/20, C07K 19/00, A61K 47/68, A61P 35/00

(54) **FC-MODIFIED IL-12 FOR LOCAL CNS DELIVERY**

(71) Applicant: Universität Zürich, 8006 Zürich (CH)
(72) Inventor: The designation of the inventor has not yet been filed
(74) Representative: Schulz Junghans Patentanwälte PartGmbB

(57) **Abstract**

The invention relates to a polypeptide comprising a crystallizable fragment (Fc) region of IgG for use in prevention or treatment of a disease affecting the central nervous system. The polypeptide is administered locally to the central nervous system. The Fc region bears a modification resulting in reduced affinity to the neonatal Fc receptor (FcRn), resulting in an increased brain to serum concentration of the polypeptide.

## Description

The present invention relates to locally delivered biological pharmaceuticals characterized by an Fc polypeptide having a lowered affinity towards the neonatal Fc receptor (FcRn) for use in neurological diseases.

### Description

### Background

Immunotherapy is one of the most promising directions in brain tumor treatment. Interleukin (IL)-12 is a pro-inflammatory cytokine and has a powerful anti-tumor effect on brain tumors in preclinical models. Based on promising preclinical results, clinical testing was rapidly initiated in the late 90s as an intravenously (i.v.) applied systemic treatment using IL-12. However, a phase II clinical trial reported severe adverse events, with 12 out of 17 patients hospitalized and two patients dead. These adverse effects have since been attributed to the rapid induction of high systemic levels of interferon (IFN)-γ, an IL-12 downstream effector cytokine.

Given the toxicity of systemically applied IL-12 and the need for a high concentration at the tumor site, a tight control over IL-12 levels in the tissue is a mandatory prerequisite of clinical applications. Local administration to the brain has recently become possible by using novel neurosurgical techniques, such as convection enhanced delivery (CED). Local intracranial delivery does however not preclude subsequent systemic leakage.

Murine IL-12Fc, a single chain fusion protein of IL-12 and the crystallisable fragment (Fc) of immunoglobulin G, shows increased pharmacostability, bioavailability and a reduced passive leakage from the brain compared to unmodified recombinant IL-12. Following local delivery to the brain, it is however actively exported across the blood brain barrier (BBB) by the neonatal Fc receptor (FcRn), a receptor that mediates export of all proteins comprising an Fc region from the cerebrospinal fluid. FcRn is also active in endothelial cells and in red pulp macrophages, where it prevents degradation and prolongs serum half-life live of Fc containing molecules and serum albumin. Compared to unmodified IL-12, IL-12Fc thus shows an increased systemic accumulation.

Based on the above mentioned state of the art, the objective of the present invention is to provide means and methods to extend the therapeutic window of pharmaceuticals that are locally delivered to the brain and preventing both export from the brain and systemic accumulation, thereby increasing the brain-to-serum ratio. This objective is attained by the claims of the present specification.

### Terms and definitions

In the context of the present specification, the term *crystallizable fragment (Fc) region* refers to a fraction of an IgG antibody comprising two identical heavy chain fragments covalently linked by disulfide bonds or to a single heavy chain fragment. The heavy chain fragments are comprised of constant domains (a C_{H}2 and a C_{H}3 domain in IgG antibody isotypes).

In the context of the present specification, the EU numbering system (Edelman et al. Proceedings of the National Academy of Sciences of the United States of America (1969) 63(1):78-85) is used for the numbering of amino acid residues in the Fc region. The EU numbering scheme is a widely adopted standard for numbering the residues in an antibody in a consistent manner.

Amino acid sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids.

In the context of the present specification, *FcRn^{tg}* relates to a mouse strain lacking functional murine FcRn and carrying a transgene for expression of the human FcRn α-chain under the control of natural human regulatory elements described by the allele symbol Tg(FCGRT)32Dcr.

In the context of the present invention, an *IL-12 polypeptide* is a polypeptide having an amino acid sequence comprising the sequence of p35 (Uniprot ID 29459) or a functional homologue thereof, and comprising the sequence of p40 (Uniprot ID29460) or a functional homologue thereof. In one embodiment, the IL-12 polypeptide has an amino acid sequence comprising both p35 and p40 sequences or homologues thereof as part of the same continuous amino acid chain. In said continuous amino acid chain only the N-terminal polypeptide (p40) functional homologue retains the signal peptide. In another embodiment, the IL-12 polypeptide comprises two distinct amino acid chains, one comprising the p35 sequence and another one comprising the p40 sequence, both having individual signal peptides. The IL-12 polypeptide has a biological activity of IL-12. A biological activity of IL-12 in the context of the present invention comprises the stimulation of NK or T cells by said IL-12 polypeptide, most prominently the stimulation of T effector cells acting through perforin.

In the context of the present specification, the terms sequence *identity and percentage of sequence identity* refer to the values determined by comparing two aligned sequences. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. One such example for comparison of nucleic acid sequences is the BLASTN algorithm that uses the default settings: Expect threshold: 10; Word size: 28; Max matches in a query range: 0; Match/Mismatch Scores: 1.-2; Gap costs: Linear. Unless otherwise stated, sequence identity values provided herein refer to the value obtained using the BLAST suite of programs (Altschul et al., J. Mol. Biol. 215:403-410 (1990)) using the above identified default parameters for protein and nucleic acid comparison, respectively.

In the context of the present specification, an Fc region with the designation "NHQ" refers to an Fc region in which the positions 253, 310 and 435 (as specified by the EU numbering system) comprise the indicated amino acid residues, in other words: N at position 253, H at position 310 and Q at position 435. This corresponds to an Fc region carrying two mutations: I253N and H435Q. Accordingly, an Fc region with the designation "IAQ" refers to an Fc region having I at position 253, A at position 310 and Q at position 435 (i.e. an Fc region carrying the mutations H310A and H435Q). Table 1 lists several examples of modified Fc regions.

### Detailed description of the invention

### IL-12Fc fusion polypeptide for use in treatment

A first aspect of the invention provides a fusion polypeptide comprising IL-12 and a crystallizable fragment (Fc) region of IgG, for use in prevention or treatment of a disease affecting the central nervous system. The Fc region bears a modification resulting in reduced affinity to the neonatal Fc receptor (FcRn). The polypeptide is administered to the brain.

Administration to the brain can be effected by intracranial delivery. Intracranial delivery may be continuous or intermittent or nonrecurring. The expression "administration to the brain" is also meant to include rinsing of a resection cavity following an operation. Administration may be intrathecal or intraparenchymal.

The modification of the Fc region results in a decreased serum to brain concentration ratio of the polypeptide. A decreased serum to brain concentration has the advantage that a high local concentration can be achieved within the brain, while negative side effects due to high systemic concentrations are prevented.

In certain embodiments, the serum or plasma to brain concentration ratio of the polypeptide is below a predetermined threshold. The predetermined threshold is selected from
a. at most 2/3 of the serum or plasma to brain concentration ratio of the same polypeptide comprising a non-modified Fc region, particularly IL-12Fc WT, or
b. at most 1/8 of the serum or plasma to brain concentration ratio of the same polypeptide neither comprising an Fc region nor peptide linkers, particularly rhIL-12
measurable 24 h after intracranial bolus injection into the striatum of FcRn^{tg} mice.

The measurement is performed 24 h after intracranial bolus injection into the striatum of FcRn^{tg} mice with 1 µl/min of 1 µg using a blunt end 26s G Hamilton syringe or CED (using a 27 G blunt-end needle with a 1 mm step at the tip made of fused silica with internal diameter of 0.1 mm and wall thickness of 0.0325 mm and a ramp-up injection regimen of 0.2 µl/minute for 5 minutes, 0.5 µl/minute for 4 minutes and 0.8 µl/minute for 2.5 minutes; total volume 5 µl, total amount 1 µg).

The fusion polypeptide according to the first aspect of the invention has a lower serum to brain concentration ratio than IL-12 linked to a non-modified Fc region (IL-12Fc WT). IL-12Fc WT has a long serum half-life live due to FcRn mediated recycling in the circulation.

The fusion polypeptide according to the first aspect of the invention has a lower serum to brain concentration ratio than rhIL-12, which shows high passive leakage from the brain.

In certain embodiments, the reduced affinity of said polypeptide to FcRn is characterized by a dissociation constant (K_{D}) selected from
a. a K_{D} that is at least 2x increased compared to a K_{D} characterizing binding of FcRn to the same polypeptide comprising a non-modified Fc region, and
b. a K_{D} that is at least 1.5x increased compared to a K_{D} characterizing binding of FcRn to the same polypeptide comprising a differently modified Fc region, namely one mutant selected from IAQ (bearing the mutations H310A and H435Q) and AAA (bearing the mutations I253A, H310A and H435A)

In certain embodiments, the K_{D} is at least 3x increased compared to a K_{D} characterizing binding of FcRn to the same polypeptide comprising a non-modified Fc region. In certain embodiments, the K_{D} is at least 4x increased compared to a K_{D} characterizing binding of FcRn to the same polypeptide comprising a non-modified Fc region. In certain embodiments, the K_{D} is at least 5x increased compared to a K_{D} characterizing binding of FcRn to the same polypeptide comprising a non-modified Fc region.

In certain embodiments, the K_{D} is at least 2x increased compared to a K_{D} characterizing binding of FcRn to the same polypeptide comprising said differently modified Fc region. In certain embodiments, the differently modified Fc region is an Fc region having I at position 253, A at position 310 and Q at position 435 (IAQ). In certain embodiments, the differently modified Fc region is an Fc region having A at position 253, A at position 310 and A at position 435 (AAA).

In certain embodiments, the intracranial delivery is effected by convection enhanced delivery (CED) or a variation thereof. CED refers to a technique that allows drugs to be delivered directly to the brain (-tumor) parenchyma. The CED procedure involves a minimally invasive surgical exposure of the brain, followed by placement of small diameter catheters directly into the brain, thereby bypassing the blood-brain-barrier. The main difference to regular bolus injection and diffusion driven infusion regimens is a pressure gradient that is created via ramping up the injection until bulk flow within the tissue is reached. Now the duration rather than the infusion rate determines the range of tissue reached. This approach allows for delivery of macromolecular drugs that would not normally enter the brain to effectively reach high concentrations within the brain (tumor) tissue.

In certain embodiments, the intracranial delivery is effected by intrathecal delivery. Intrathecal administration refers to direct administration of drugs into the cerebrospinal fluid (CSF). Intrathecal administration is defined as substance application below the subarachnoid membrane into the subarachnoid space in the brain (e.g. via the ommaya reservoir) or in the spinal cord. Non-limiting examples are intrathecal delivery to treat leptomeningeal carcinomatosis and primary Her2/neu positive brain tumors as well as CD20 positive CNS lymphoma and intraocular lymphoma, using trastuzumab or rituximab, respectively. Another example is intrathecal application of anti-NogoA antibodies for the treatment of acute spinal cord injury, multiple sclerosis or stroke. This approach allows for delivery of macromolecular drugs that would not normally enter the brain to effectively reach high concentrations at the leptomeninges or brain parenchyma.

In certain embodiments, the intracranial delivery is effected by intracerebroventricular delivery of said polypeptide. Intracerebroventricular administration refers to direct administration of drugs into the cerebrospinal fluid (CSF) by means of a cathether into the ventricular lumen.

In certain embodiments, the intracranial delivery is effected by in situ production of said polypeptide. In situ production relates to local production of the polypeptide exclusively or virtually exclusively within the brain or the brain tumor. By way of non-limiting example, local production may originate from DNA formulations, mRNA, modified mRNA, self-replicating mRNA, viral vectors, encapsulated modified producer cells or modified T cells. A spatial control over the local production can be achieved by local delivery of the molecules or vectors encoding the polypeptide or by local activation the production of the polypeptide. Local production via local delivery of the molecules or vectors encoding the polypeptide and subsequent local activation of the production of the polypeptide can be achieved via local or systemic administration of agents acting as transcriptional derepressors or transcriptional activators of conditional expression cassettes. Examples include but are not limited to ecdysone receptor/invertebrate retinoid x receptor-based inducible gene expression systems or tetracycline-regulated transcriptional modulators.

In certain embodiments, the intracranial delivery is effected by systemic delivery of cells modified to produce said polypeptide with homing capabilities to the tumor or CNS. The polypeptide may be produced in a constitutive or inducible manner. Examples include but are not limited to modified T cells or mesenchymal stem cells.

In certain embodiments, the intracranial delivery is effected by release from implanted slow-release / extended-release / sustained-release / controlled-release formulations. In the context of the present specification, such formulations relate to dosage forms designed to release a drug at a predetermined rate in order to maintain a constant drug concentration for a specific period of time with minimum side effects. The skilled person is aware of a variety of suitable formulations. Non-limiting examples are liposomes, drug-polymer conjugates, hydrogels, wavers or coated nanoparticles.

In certain embodiments, the intracranial delivery is effected by intranasal delivery of said polypeptide.

In certain embodiments, the intracranial delivery is effected by receptor mediated transcytosis of said polypeptide. A non-limiting example is a bispecific construct binding to TfR as well as a target found in the diseased brain parenchyma, particularly Aβ plaques in Alzheimer's Disease (AD).

### Diseases affecting the central nervous system

In certain embodiments, the disease affecting the central nervous system is a malignant disease.

In certain embodiments, the disease affecting the central nervous system is a glioma. In certain embodiments, the disease affecting the central nervous system is a high grade glioma (HGG).

In certain embodiments, the disease affecting the central nervous system a secondary brain tumor, also known as brain metastases.

In certain embodiments, the disease affecting the central nervous system is ischemic brain injury or cerebral infarction, stroke, brain hypoxia-ischemia, intracranial embolism or intracranial thrombosis.

In certain embodiments, the disease affecting the central nervous system is epilepsy, traumatic brain injury.

In certain embodiments, the disease affecting the central nervous system is a spinal cord injury, dementia, Parkinson's Disease (PD), Lewy Bodies, Alzheimer's Disease (AD), frontotemporal dementia (FTD), familial frontotemporal dementia (FTD), or Amyotrophic Lateral Sclerosis (ALS).

In certain embodiments, the disease affecting the central nervous system is a transmissible spongiform encephalopathy, particularly Creutzfeld Jakob Disease (CJD), Kuru, Scrapie, Bovine spongiform encephalopathy (BSE). In certain embodiments, the disease affecting the central nervous system is a hereditary disorder, particularly Cerebral Autosomal Dominant Arteriopathy with Subcortical Infarcts and Leukoencephalopathy (CADASIL) In certain embodiments, the disease affecting the central nervous system is a hereditary disorder, particularly Huntington's Disease. In certain embodiments, the disease affecting the central nervous system is a hereditary disorder, particularly Autism, autism spectrum disorders (ASD), e.g. Asperger Syndrome.

In certain embodiments, the disease affecting the central nervous system is hereditary Leukodystrophy, particularly metachromatic leukodystrophy, Krabbe Disease, Canavan Disease, X-linked Adrenoleukodystrophy, Alexander Disease. In certain embodiments, the disease affecting the central nervous system is a hereditary metabolic disorder, particularly Tay-Sachs Disease or Wilson Disease.

In certain embodiments, the disease affecting the central nervous system is a psychiatric disorder, particularly amnesia, attention-deficit hyperactivity disorder, psychosis, anxiety disorders, bipolar disorders, depression, mania, intellectual developmental disorder, global developmental delay, post-traumatic stress disorder, acute stress disorder, dissociative disorders.

In certain embodiments, the disease affecting the central nervous system is epilepsy. In certain embodiments, the disease affecting the central nervous system is autoimmune encephalitis. In certain embodiments, the disease affecting the central nervous system is multiple sclerosis. In certain embodiments, the disease affecting the central nervous system is neuromyelitis optica (NMO). In certain embodiments, the disease affecting the central nervous system is autoimmune encephalitis, particularly anti-NMDAR encephalitis, limbic encephalitis, LGI1/CASPR2-antibody encephalitis, hashimoto's encephalopathy, acute Disseminated Encephalomyelitis (ADEM), Binswanger's Disease (Subcortical Leukoencephalopathy), Rasmussen's Encephalitis.

In certain embodiments, the disease affecting the central nervous system is infectious encephalomyelitis caused by viruses, particularly rabies virus, human herpes viruses, rash-causing viruses, insect-borne viruses, tick-borne viruses, human immunodeficiency virus (HIV).

In certain embodiments, the disease affecting the central nervous system is infectious encephalomyelitis caused by bacteria or infectious encephalomyelitis caused by parasites.

In certain embodiments, the disease affecting the central nervous system is progressive multifocal leukoencephalopathy (PML) caused by JC polyomavirus (usually abbreviated as JCPyV or JCV)

In certain embodiments, the disease affecting the central nervous system is postinfectious encephalomyelitis.

In certain embodiments, the disease affecting the central nervous system is neovascular age-related macular degeneration (wet AMD) and diabetic macular edema or retinitis pigmentosa.

In certain embodiments, the Fc region is a chimeric Fc region comprising a human or humanized amino acid sequence.

In certain embodiments, the Fc region is a human or humanized Fc region.

In certain embodiments, the Fc region bears a mutation at position 253 relative to SEQ ID NO 1. In certain embodiments, the Fc region bears the mutation I253A. In certain embodiments, the Fc region bears the mutation I253N.

In certain embodiments, the Fc region bears a mutation at position 435 relative to SEQ ID NO 1. In certain embodiments, the Fc region bears the mutation H435Q.

In certain embodiments, the Fc region does not bear a mutation at position 435 relative to SEQ ID NO 1. Thus, the Fc region bears a H at position 435.

In certain embodiments, the Fc region does not bear a mutation at position 310 relative to SEQ ID NO 1. Thus, the Fc region bears a H at position 310.

In certain embodiments, the Fc region comprises
- the mutations I253A and H435Q, and an H at position 310 (AHQ);
- the mutations I253N and H435Q, and an H at position 310 (NHQ);
- the mutations I253A, H310A and H435Q (AAQ);
- the mutations I253N, H310A and H435Q (NAQ);
- the mutation I253A and an H at position 310 and 435 (AHH);
- the mutation I253N and an H at position 310 and 435 (NHH);
- the mutations I253A and H310A, and an H at position 435 (AAH);
- the mutations I253N and H310A, and an H at position 435 (NAH);
- the mutations I253N, H310A and H435A (NAA);
- the mutations I253N, H310A and H435E (NAE);
- the mutations I253A, H310A and H435A (AAA); or
- the mutations I253A, H310A and H435E (AAE).

In certain embodiments, the Fc region comprises
- the mutations I253N and H435Q, and an H at position 310 (NHQ);
- the mutations I253A, H310A and H435Q (AAQ);
- the mutations I253N, H310A and H435Q (NAQ);
- the mutations I253N, H310A and H435E (NAE);or
- the mutations I253A, H310A and H435E (AAE).

In certain embodiments, the Fc region comprises the mutations I253N and H435Q, and an H at position 310 (NHQ).

In certain embodiments, the Fc region comprises the mutations I253A, H310A and H435Q (AAQ). In certain embodiments, the Fc region comprises the mutations I253N, H310A and H435Q (NAQ). In certain embodiments, the Fc region comprises the mutations I253N, H310A and H435E (NAE). In certain embodiments, the Fc region comprises the mutations I253A, H310A and H435E (AAE).

In certain embodiments, the Fc region is or comprises a sequence characterized by SEQ ID NO 002 (IAQ), SEQ ID NO 003 (AHQ), SEQ ID NO 004 (NHQ), SEQ ID NO 005 (AAQ), SEQ ID NO 006 (NAQ), SEQ ID NO 007 (AHH), SEQ ID NO 008 (NHH), SEQ ID NO 009 (AAH), SEQ ID NO 010 (NAH), SEQ ID NO 011 (NAA), SEQ ID NO 012 (NAE), SEQ ID NO 013 (AAA) or SEQ ID NO 014 (AAE).

In certain embodiments, the Fc region is or comprises a sequence characterized by SEQ ID NO 004 (NHQ), SEQ ID NO 005 (AAQ), SEQ ID NO 006 (NAQ), SEQ ID NO 012 (NAE) or SEQ ID NO 014 (AAE). In certain embodiments, the Fc region is or comprises a sequence characterized by SEQ ID NO 004 (NHQ). In certain embodiments, the Fc region is or comprises a sequence characterized by SEQ ID NO 006 (NAQ). In certain embodiments, the Fc region is or comprises a sequence characterized by SEQ ID NO 012 (NAE). In certain embodiments, the Fc region is or comprises a sequence characterized by SEQ ID NO 014 (AAE).

### Polypeptide comprising a crystallizable fragment (Fc) region for use in treatment

A broader aspect of the invention provides a polypeptide comprising a crystallizable fragment (Fc) region of IgG, for use in prevention or treatment of a disease. The Fc region bears a modification resulting in reduced affinity to the neonatal Fc receptor (FcRn), and the polypeptide is delivered by local administration to the tissue affected by the disease.

In certain embodiments, the polypeptide is delivered to the eye by intraocular administration.

In certain embodiments, the polypeptide is delivered to a joint by intraarticular administration.

In certain embodiments, the polypeptide is delivered to the lungs via inhalation.

Following local administration, the reduced affinity to FcRn ensures that transport into the circulation and systemic enrichment is reduced, thereby reducing any systemic toxic side effects of the polypeptide.

In certain embodiments, the polypeptide is selected from
a. a fusion protein comprising
   i. an effector polypeptide and
   ii. said Fc region; or
b. an antibody or antibody-like molecule comprising or linked to said Fc region.

Further embodiments of the polypeptide comprising a crystallizable fragment (Fc) region for use in treatment can be found in the "items" section below.

### Targeting the PD-1/PD-L1 axis for use in treatment

Another aspect of the invention provides an antibody or antibody-like molecule specifically binding to programmed cell death protein 1 (PD-1) or programmed death-ligand 1 (PD-L1) for use in prevention or treatment of a disease affecting the central nervous system. The antibody or antibody-like molecule comprises an Fc region bearing a modification resulting in reduced affinity to the neonatal Fc receptor (FcRn). The antibody or antibody-like molecule is administered to the brain.

### anti-OX40 for use in treatment

Another aspect of the invention provides an antibody or antibody-like molecule specifically binding to tumor necrosis factor receptor superfamily, member 4 (TNFRSF4), also known as CD134, OX40 or OX40 receptor for use in prevention or treatment of a disease affecting the central nervous system. The antibody or antibody-like molecule comprises an Fc region bearing a modification resulting in reduced affinity to the neonatal Fc receptor (FcRn). The antibody or antibody-like molecule is administered to the brain.

### anti-CD47 for use in treatment

Another aspect of the invention provides an antibody or antibody-like molecule specifically binding to CD47, also known as integrin associated protein (IAP) for use in prevention or treatment of a disease affecting the central nervous system. Yet another aspect of the invention provides a ligand of CD47, particularly SIRPα or thrombospondin-1 (TSP-1) fused with an Fc region. The antibody or antibody-like molecule or Fc-fusion molecule comprises an Fc region bearing a modification resulting in reduced affinity to the neonatal Fc receptor (FcRn). The antibody or antibody-like molecule or Fc-fusion molecule is administered to the brain.

### anti-Nogo-A for use in treatment

Another aspect of the invention provides an antibody or antibody-like molecule specifically binding to Nogo-A for use in prevention or treatment of a disease affecting the central nervous system. Yet another aspect of the invention provides a ligand of Nogo-A, particularly the Nogo-66 Receptor also known as Nogo Receptor 1 (NgR1) fused with an Fc region. The antibody or antibody-like molecule or Fc-fusion molecule comprises an Fc region bearing a modification resulting in reduced affinity to the neonatal Fc receptor (FcRn). The antibody or antibody-like molecule or Fc-fusion molecule is administered to the brain.

### T cell engaging bispecific antibodies for use in treatment

Another aspect of the invention provides T cell engaging bispecific antibodies, bispecific antibodies or antibody-like molecules specifically binding to a tumor associated antigen (TAA) on cancer cells and at the same time to CD3 on T cells providing T cell receptor independent polyclonal activation if bound to the TAA for use in prevention or treatment of a disease affecting the central nervous system. Yet another aspect of the invention provides a bispecific antibody or antibody-like molecule specifically binding to PD-L1 and at the same time to 4-1BB on T cells. An even further aspect of the invention provides a bispecific antibody or antibody-like molecule specifically binding to PD-L1 and at the same time to CD28 on T cells. The antibody or antibody-like molecule or Fc-fusion molecule comprises an Fc region bearing a modification resulting in reduced affinity to the neonatal Fc receptor (FcRn). The antibody or antibody-like molecule or Fc-fusion molecule is administered to the brain.

### Armed antibodies and targeted antibodies for use in treatment

Another aspect of the invention provides an armed antibody or antibody-like molecule specifically binding to a tumor associated antigen (TAA) on cancer cells or to an antigen present in the tumor vasculature or an antigen present in the necrotic core of the tumor. Said antibody or antibody-like molecule also carries an effector molecule, particularly a cytokine, a radioactive isotope or a cytotoxic substance for use in prevention or treatment of a disease affecting the central nervous system. The armed antibody or antibody-like molecule or Fc-fusion molecule comprises an Fc region bearing a modification resulting in reduced affinity to the neonatal Fc receptor (FcRn). The antibody or antibody-like molecule or Fc-fusion molecule is administered to the brain.

### Tumor conditional or tissue conditional antibodies

Another aspect of the invention provides an antibody or antibody-like molecule that binds specifically to a first tumor associated antigen (TAA) on cancer cells or to an antigen present in the tumor microenvironment or an antigen present in the necrotic core of the tumor or an antigen present in the target tissue. Said antibody or antibody-like molecule also comprises a second effector molecule, particularly a cytokine or is a bispecific or multispecific antibody with at least one more antibody or antibody-like molecule binding a second antigen different from the first for use in prevention or treatment of a disease affecting the central nervous system. In such a construct, the second antibody or antibody-like domain is shielded and cannot bind its target antigen. The shielding domain is connected to the second antibody or antibody-like construct via a protease sensitive linker peptide that will be cleaved in the tumor or target tissue by proteases predominantly or exclusively found there. The shielding domain can be the antibody or antibody-like molecule binding the first antigen. Upon cleavage of the shielding domain in the target tissue or tumor microenvironment the second antibody or antibody-like molecule will bind its target antigen or, in case of a cytokine or chemokine, will bind to its receptor. The tumor conditional or tissue conditional antibody or antibody-like molecule or Fc-fusion molecule comprises an Fc region bearing a modification resulting in reduced affinity to the neonatal Fc receptor (FcRn). The antibody or antibody-like molecule or Fc-fusion molecule is administered to the brain.

The skilled person is aware that in the case of an antibody, the antibody itself already comprises an Fc region. In the case of an antibody-like molecule, the antibody-like molecule or Fc-fusion molecule is linked to an Fc region.

Further embodiments of the antibody or antibody-like molecule or Fc-fusion molecule comprising a crystallizable fragment (Fc) region for use in treatment can be found in the "items" section below.

### Polypeptide comprising Fc region with a reduced affinity to FcRn (increased K_{D})

A second aspect of the invention provides a polypeptide comprising a Fc region of IgG, wherein said Fc region bears a modification resulting in reduced affinity to the neonatal Fc receptor (FcRn) compared to the affinity of the same polypeptide comprising a non-modified Fc region.

In certain embodiments, the reduced affinity of said polypeptide to FcRn is characterized by a dissociation constant (K_{D}) selected from
a. a K_{D} that is at least 2x increased compared to a K_{D} characterizing binding of FcRn to the same polypeptide comprising a non-modified Fc region, and
b. a K_{D} that is at least 1.5x increased compared to a K_{D} characterizing binding of FcRn to the same polypeptide comprising a differently modified Fc region, namely one mutant selected from IAQ (bearing the mutations H310A and H435Q) and AAA (bearing the mutations I253A, H310A and H435A)

In certain embodiments, the K_{D} is at least 3x increased compared to a K_{D} characterizing binding of FcRn to the same polypeptide comprising a non-modified Fc region. In certain embodiments, the K_{D} is at least 4x increased compared to a K_{D} characterizing binding of FcRn to the same polypeptide comprising a non-modified Fc region. In certain embodiments, the K_{D} is at least 5x increased compared to a K_{D} characterizing binding of FcRn to the same polypeptide comprising a non-modified Fc region.

In certain embodiments, the K_{D} is at least 2x increased compared to a K_{D} characterizing binding of FcRn to the same polypeptide comprising said differently modified Fc region.

In certain embodiments, the polypeptide is selected from
a. a fusion protein comprising
   i. an effector polypeptide and
   ii. said Fc region; or
b. an antibody or antibody-like molecule comprising or linked to said Fc region.

The skilled person is aware that in the case of an antibody, the antibody itself already comprises an Fc region. In the case of an antibody-like molecule, the antibody-like molecule is linked to an Fc region.

In certain embodiments, the effector polypeptide has the function of
a. a cytokine or hormone or growth factor,
b. a cytokine receptor or hormone receptor or growth factor receptor, or
c. a metabolite
and is known to have a therapeutic or preventive effect on a disease, in particular on a disease affecting the central nervous system.

In certain embodiments, the effector polypeptide is able to specifically bind to the extracellular matrix (ECM) and is known to have a therapeutic or preventive effect on a disease, in particular on a disease affecting the central nervous system. In certain embodiments, the effector polypeptide is able to specifically bind to RNA and is known to have a therapeutic or preventive effect on a disease, in particular on a disease affecting the central nervous system.

In certain embodiments, the effector polypeptide is selected from the group comprising IL-12, IL-10, IL-2, IL-7, IFNα, IFNβ, IFNγ, IL-15, TNFα, CTLA-4, TGFβ, TGFβRII, GDNF, IL-35, CD95, IL-1RA, IL-4, IL-13, IL-33, IL-23, SIRPα, G-CSF, GM-CSF, OX40L, CD80, CD86, GITRL, 4-1BBL, EphrinA1, EphrinB2, EphrinB5, BDNF, C9orf72, NRTN, ARTN, PSPN, CNTF, TRAIL, IL-4, IL-3, IL-1, IL-5, IL-8, IL-18, IL-21, CCL5, CCL21, CCL10, CCL16, CX3CL1, CXCL16 in particular said effector polypeptide is IL-12.

In certain embodiments, the antibody or antibody-like molecule is selected from an antibody or antibody-like molecule specifically binding to PD-L1, TNFα, Histone, IFNγ, CXCL10, CTLA4, PD-1, CD3, OX40, CD25, CD28, TREM2, IL-6, CX3CR1, Nogo-A, CD27, IL-12, IL-12Rb1, IL-23, IL-17, CD47, TGFβ, EGFR, EGFRvIII, Her2, PDGFR, TGFR, FGFR, IL-4RA, TfR, LfR, IR, LDL-R, LRP-1, CD133, CD111, VEGFR, VEGF-A, Ang-2, IL-10, IL-10R, IL-13Rα2, α-synuclein, CSF1R, G-CSF, GM-CSF, GITR, TIM-3, LAG-3, TIGIT, BTLA, VISTA, CD96, 4-1BB, CCL2, IL-1 or IL-1R, EphA2, EphA3, EphB2, EphB3, EphB4, LINGO-1, L1CAM, NCAM, SOD-1, SIGMAR-1, SIGMAR-2, TDP-43, Aβ, Tau, IFNα, IFNβ, TRPM4, ASIC1, VGCCs, CB₁, TTR, HTT, JCV, C9orf72 in an agonistic or antagonistic fashion.

An antibody or antibody-like molecule according to the above aspect of the invention may be an antibody-like molecule derived from the recognition site of a physiological ligand of PD-1 or PD-L1 or PD-L2 or a full antibody. Such antibody or antibody-like molecule competes with the physiological ligand for binding to PD-1 or PD-L1 or PD-L2, respectively. Particularly, a non-agonist PD-1 antibody or antibody-like molecule or non-agonist PD-L1 antibody or antibody-like molecule or non-agonist PD-L2 antibody or antibody-like molecule does not lead to attenuated T cell activity when binding to PD-1, on the surface on a T-cell.

In some embodiments, non-agonist PD-1 antibodies or antibody-like molecules used in the present invention are able, when bound to PD-1, to sterically block interaction of PD-1 with its binding partners PD-L1 and/or PD-L2.

In some embodiments, said non-agonist PD-1 antibody or antibody-like molecule is a gamma immunoglobulin binding to PD-1, without triggering the physiological response of PD-1 interaction with its binding partners PD-L1 and/or PD-L2.

In some embodiments, said non-agonist PD-L1 (PD-L2) antibody or antibody-like molecule is a gamma immunoglobulin binding to PD-L1 (PD-L2), without triggering the physiological response of PD-1 interaction with its binding partners PD-L1 and/or PD-L2.

Non-limiting examples for a PD-1 antibody are the clinically approved antibodies pembrolizumab (CAS No. 1374853-91-4) and nivolumab (CAS No. Number 946414-94-4)

Non-limiting examples for a PD-L1 antibody are the clinically approved antibodies atezolizumab (CAS No. 1380723-44-3), durvalumab (CAS No. 1428935-60-7) and avelumab (CAS No. 1537032-82-8).

Non-limiting examples for a PD-1 / PD-L1 or PD-L2 antibody currently undergoing clinical development are the antibodies MDX-1105/BMS-936559 or AMP-224. A non-limiting example of an antibody specifically binding to IL-12/23 is Ustekinumab (CAS No. 815610-63-0).

In certain embodiments, the antibody or antibody-like molecule is an antibody specifically binding to PD-L1.

In some embodiments, agonistic OX40 antibodies or antibody-like molecules used in the present invention are able to trigger a signalling cascade in OX40 expressing cells upon binding to OX40 and in the absence of OX40 ligand.

Non-limiting examples for an OX40 antibody are the antibodies PF-04518600/PF-8600m BMS-986178, GSK3174998, MOXR0916, INCAGN01949, Tavolimab/MEDI0562, currently undergoing clinical development.

In certain embodiments, the antibody or antibody-like molecule is an antibody specifically binding to OX40.

In some embodiments, antibodies or antibody-like molecules used in the present invention are able to block the interaction between CD47 and SIRPα signals which prevent phagocytosis of cancer cells.

Non-limiting examples of CD47 blocking antibodies or SIRPα fusion proteins are Hu5F9-G4, CC-90002/INBRX-103, IBI188, OSE-172, NI-1801, DSP107, TTI-622, TTI-621, ALX148, and SRF231.

In certain embodiments, the antibody or antibody-like molecule is an antibody specifically binding to Nogo-A.

In certain embodiments, the antibody or antibody-like molecule is a bispecific construct able to bind two antigens at the same time.

In certain embodiments, the antibody or antibody-like molecule is an antibody directed against histones present in the necrotic core of tumors, which is armed with IL-12 or IL-2. In certain instances armed antibodies are immunocytokines. Non-limiting examples of armed antibodies as immunocytokines are NHS-IL-12, NHS-IL2LT, Hu14.18-IL2, HuKS-IL2, huBC1-IL-12.

In certain embodiments, the Fc region bears a mutation at position 253. In certain embodiments, the Fc region bears the mutation I253A. In certain embodiments, the Fc region bears the mutation I253N.

In certain embodiments, the Fc region bears a mutation at position 435. In certain embodiments, the Fc region bears the mutation H435Q.

In certain embodiments, the Fc region does not bear a mutation at position 435. Thus, the Fc region bears a H at position 435.

In certain embodiments, the Fc region does not bear a mutation at position 310. Thus, the Fc region bears a H at position 310.

In certain embodiments, the Fc region comprises
- the mutations I253A and H435Q, and an H at position 310 (AHQ);
- the mutations I253N and H435Q, and an H at position 310 (NHQ);
- the mutations I253A, H310A and H435Q (AAQ);
- the mutations I253N, H310A and H435Q (NAQ);
- the mutation I253A and an H at position 310 and 435 (AHH);
- the mutation I253N and an H at position 310 and 435 (NHH);
- the mutations I253A and H310A, and an H at position 435 (AAH);
- the mutations I253N and H310A, and an H at position 435 (NAH);
- the mutations I253N, H310A and H435A (NAA);
- the mutations I253N, H310A and H435E (NAE);
- the mutations I253A, H310A and H435A (AAA); or
- the mutations I253A, H310A and H435E (AAE).

In certain embodiments, the Fc region comprises
- the mutations I253N and H435Q, and an H at position 310 (NHQ);
- the mutations I253A, H310A and H435Q (AAQ);
- the mutations I253N, H310A and H435Q (NAQ);
- the mutations I253N, H310A and H435E (NAE);or
- the mutations I253A, H310A and H435E (AAE).

In certain embodiments, the Fc region comprises the mutations I253N and H435Q, and an H at position 310 (NHQ).

In certain embodiments, the Fc region comprises the mutations I253A, H310A and H435Q (AAQ). In certain embodiments, the Fc region comprises the mutations I253N, H310A and H435Q (NAQ). In certain embodiments, the Fc region comprises the mutations I253N, H310A and H435E (NAE). In certain embodiments, the Fc region comprises the mutations I253A, H310A and H435E (AAE).

In certain embodiments, the Fc region is or comprises a sequence characterized by SEQ ID NO 002 (IAQ), SEQ ID NO 003 (AHQ), SEQ ID NO 004 (NHQ), SEQ ID NO 005 (AAQ), SEQ ID NO 006 (NAQ), SEQ ID NO 007 (AHH), SEQ ID NO 008 (NHH), SEQ ID NO 009 (AAH), SEQ ID NO 010 (NAH), SEQ ID NO 011 (NAA), SEQ ID NO 012 (NAE), SEQ ID NO 013 (AAA) or SEQ ID NO 014 (AAE).

In certain embodiments, the Fc region is or comprises a sequence characterized by SEQ ID NO 004 (NHQ), SEQ ID NO 005 (AAQ), SEQ ID NO 006 (NAQ), SEQ ID NO 012 (NAE) or SEQ ID NO 014 (AAE). In certain embodiments, the Fc region is or comprises a sequence characterized by SEQ ID NO 004 (NHQ). In certain embodiments, the Fc region is or comprises a sequence characterized by SEQ ID NO 006 (NAQ). In certain embodiments, the Fc region is or comprises a sequence characterized by SEQ ID NO 012 (NAE). In certain embodiments, the Fc region is or comprises a sequence characterized by SEQ ID NO 014 (AAE).

In certain embodiments of any aspect of the invention, the polypeptide comprising a modified Fc region according to the invention is used in combination with an FcRn-blocking antibody. FcRn-blocking antibodies are capable of inhibiting the binding between Fc-comprising polypeptides and FcRn, thus mimicking the technical effect of the invention. The combination with an FcRn-blocking antibody may enhance the described advantages of a polypeptide comprising a modified Fc region according to the invention.

In certain embodiments of any aspect of the invention, the Fc region is an Fc region of immunoglobulin G (IgG). IgG is a major effector molecule of the humoral immune response in man. There are four distinct subgroups of human IgG designated IgG1, IgG2, IgG3 and IgG4. The four subclasses show more than 95% homology in the amino acid sequences of the constant domains of the heavy chains, but differ with respect to structure and flexibility of the hinge region, especially in the number of inter-heavy chain disulfide bonds in this domain. The structural differences between the IgG subclasses are also reflected in their susceptibility to proteolytic enzymes, particularly papain, plasmin, trypsin and pepsin.

In certain embodiments of any aspect of the invention, the Fc region is an Fc region of IgG4. Only one isoform of human IgG4 is known. In contrast to human IgG1, IgG2 and IgG3, human IgG4 does not activate complement. Furthermore, IgG4 is less susceptible to proteolytic enzymes compared to IgG2 and IgG3.

Similarly within the scope of the present invention is a use of treating or preventing a malignant neoplastic disease, particularly a solid tissue tumor, more particularly glioma, in a patient in need thereof, comprising administering to the patient a polypeptide comprising a modified Fc region according to one of the aspects of the invention described above or a nucleic acid encoding the polypeptide or a viral vector comprising the nucleic acid encoding the polypeptide.

Similarly, a dosage form for the prevention or treatment of a malignant neoplastic disease, particularly a solid tissue tumor, more particularly glioma, is provided, comprising a polypeptide comprising a modified Fc region according to one of the aspects of the invention described above or a nucleic acid encoding the polypeptide or a viral vector comprising the nucleic acid encoding the polypeptide.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Brief description of the figures

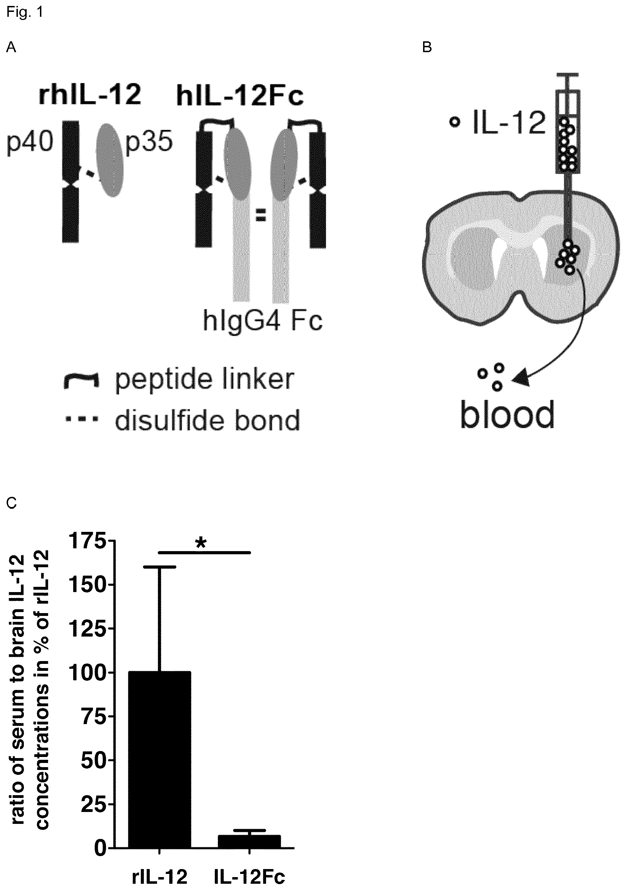

Figure 1: Human IL-12Fc has better tissue retention than IL-12. A. Schematic structure of murine IL-12Fc. rhIL-12 - recombinant human IL-12, hIL-12Fc - human IL-12Fc. IgG4 Fc - fragment crystallizable region of human IgG4. B. Schematic of the experiment. IL-12 of IL-12Fc was injected into the striatum of FcRn^{tg} mice. After 24 hours the remaining amount of injected protein was assessed in the brain and compared to the amount present in serum. C. Ratio of serum to brain IL-12 amount as assessed by ELISA. ELISA measured hIL-12 as a generic measure of IL-12Fc. Unpaired Student's t-test. **p<0.005. Mean ± SD.

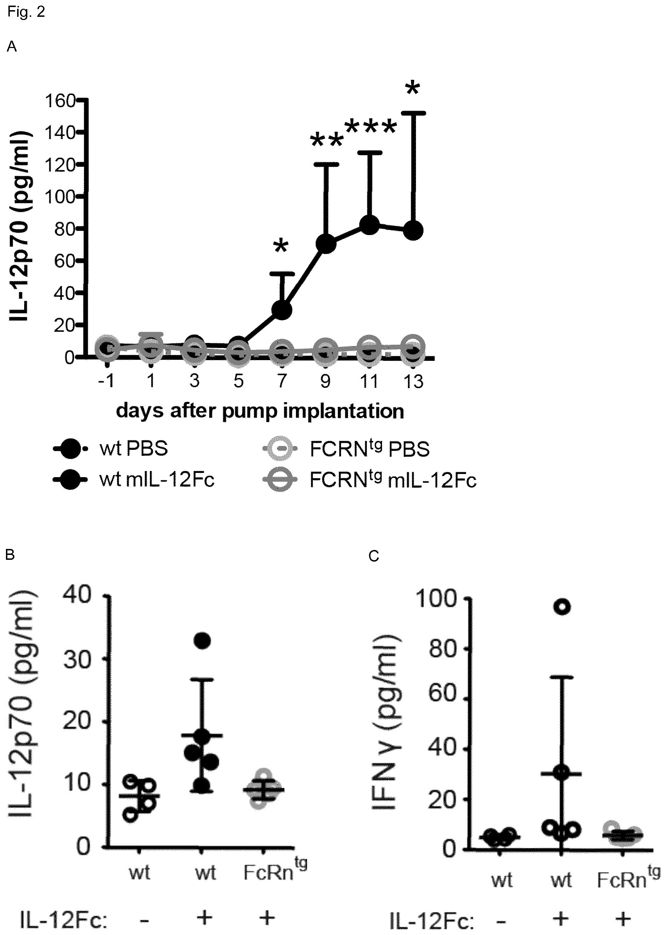

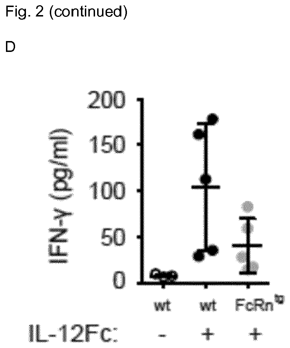

Figure 2: IL-12Fc is being exported from the brain in an FcRn-mediated fashion. A. Brain tumor-bearing wt and FcRn^{tg} mice were implanted with osmotic pumps delivering 12.5 µg/kg/day of murine IL-12Fc directly into the tumor lesion. Murine IL-12 levels measured in serum using a bead-based array. Unpaired Student's t-test of groups wt mIL-12Fc vs FcRn^{tg} mIL-12Fc. Mean ± SD. One way ANOVA with Tukey's multiple comparison test. B. Mice treated like in Fig. 2 A. Amount of IL-12 present in the circulation 24 hours after the start of the treatment as measured in serum using a bead-based array. Mean ± SD. C. Mice treated like in Fig. 2 A. Levels of IFNγ in the circulation 24 hours after the start of the treatment as measured in serum using a bead-based array. Mean ± SD. D. IFN-γ levels at day 7, experiment in A, Mean ± SD.

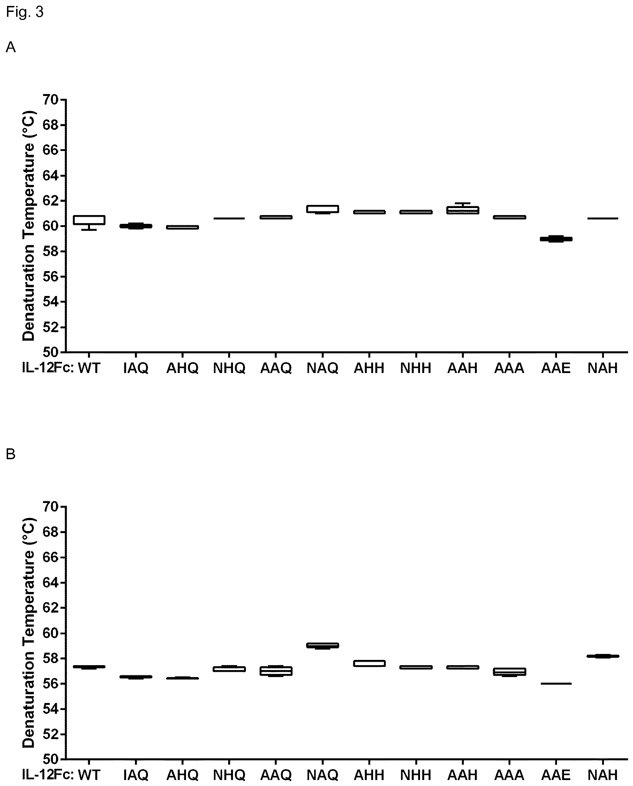

Figure 3: Protein stability measured using thermal shift assay. Protein was incubated in PBS (A) or artificial cerebrospinal fluid (aCSF, B). Five measurements per IL-12Fc variant. Whiskers represent the minimum and maximum spread.

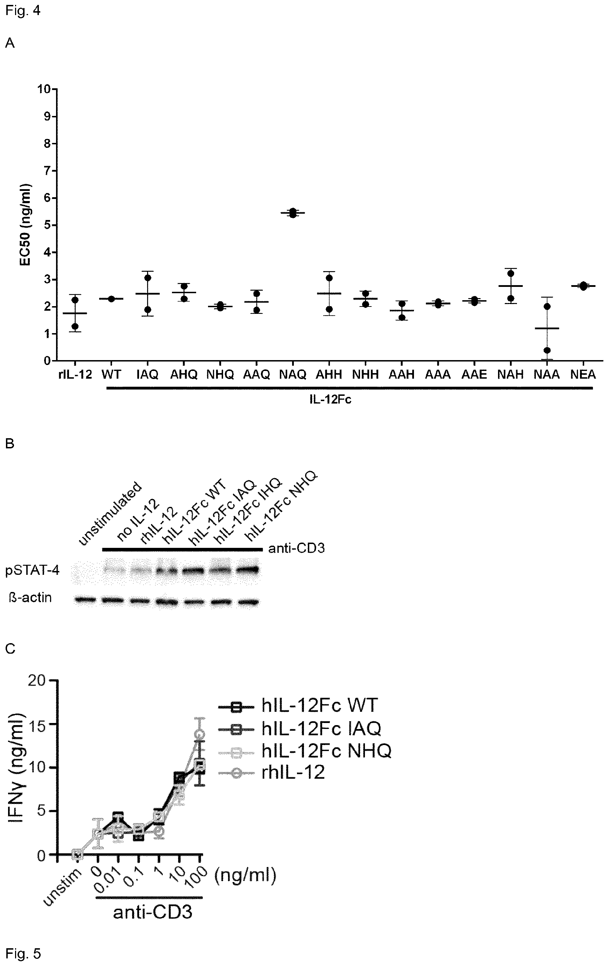

Figure 4: Mutations in the Fc fragment of IL-12Fc do not affect the biological activity of IL-12. A. Bioactivity of IL-12 measured using HEK-Blue™ IL-12 assay. EC50 - effective concentration leading to 50% of maximal signal from HEK-Blue™ IL-12 reporter cells stimulated with IL-12Fc in the range 0 to 50 ng/ml, two replicates per concentration. Measured by using the activity of the secreted alkaline phosphatase using a colorimetric method. Each point shows result from an independent experiment. Mean ± SD. B. STAT-4 phosphorylation in peripheral blood mononuclear cells (PBMCs) stimulated for 1 h with 100 ng/ml anti-CD3 and 10 ng/ml of recombinant IL-12, IL-12Fc WT or three of the variants designed for reduced FcRn affinity. Mean ± SD. C. IFNγ production by PBMCs stimulated for 24 h with 100 ng/ml anti-CD3 and indicated concentrations of recombinant IL-12, IL-12Fc WT or three of the variants designed for reduced FcRn affinity.

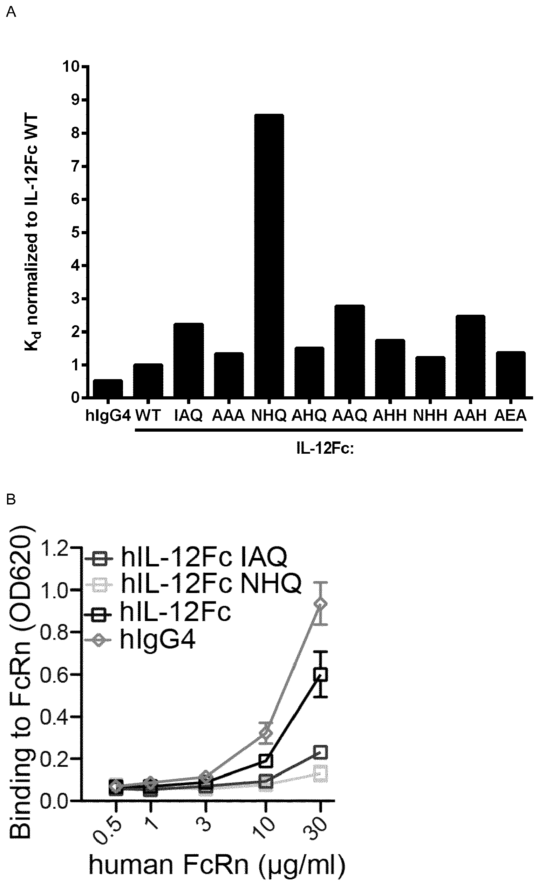

Figure 5: Human IL-12Fc variants have reduced FcRn affinity. A. Surface plasmon resonance (SPR) measurement of FcRn affinity with human recombinant FcRn immobilized on the surface and IL-12Fc variants in the liquid phase. Affinity measured at pH = 6.0. Data normalized to IL-12Fc WT. B. IL-12Fc variants binding to human FcRn. Measured by ELISA at pH = 6.0. Mean ± SD.

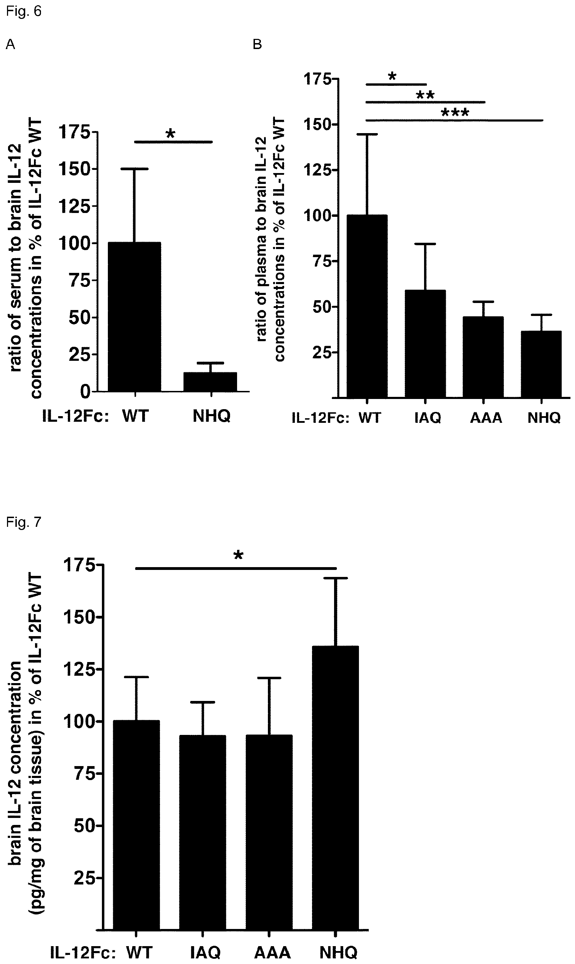

Figure 6. Ratios of the concentrations of IL-12Fc in the blood and in the injected hemisphere. A. 1 µg of IL-12Fc WT or NHQ variant were injected into the striatum of FcRn^{tg} mice. After 24 hours the amounts of IL-12 were assessed in the injected brain hemisphere and in serum by ELISA, their ratios were calculated and normalized to those for IL-12Fc WT group. 4 mice per group. Unpaired Student's t-test. *p<0.05. Mean ± SD. B. 1 µg of IL-12Fc WT, IAQ, AAA or NHQ were injected into the striatum of FcRn^{tg} mice using convection enhanced delivery (CED). After 24 hours the amounts of IL-12 were assessed in the injected brain hemisphere and in plasma by ELISA, their ratios were calculated and normalized to those for IL-12Fc WT group. 7-8 mice per group. One-way ANOVA with Tukey's multiple comparison test. Mean ± SD.
Figure 7: Brain retention after local treatment with IL-12Fc variants. FcRn^{tg} mice were injected with 1 µg of IL-12Fc WT, IAQ, AAA or NHQ into the striatum using convection enhanced delivery (CED). Amount of IL-12Fc remaining in the brain tissue was measured 6 hours after injection by ELISA and normalized to IL-12Fc WT. One-way ANOVA with Tukey's multiple comparison test. Outlier removal. Mean ± SD.

### Examples

### Example 1: Material and Methods

### Animals

C57BL/6J mice were obtained from Charles River. *mFcRn^{-l-}hFcRn^{tg(32)}* (FcRn^{tg}) mice were obtained from The Jackson Laboratory (stock number 014565). All animals were kept in house according to institutional guidelines under specific pathogen-free (SPH) conditions at a 12h light/dark cycle with food and water provided ad libitum.

### Tumor cell lines

GL-261 cells were provided by A. Fontana, Experimental Immunology, University of Zurich, Zurich, Switzerland and cultured in DMEM supplemented with 10% heat inactivated fetal calf serum and L-glutamine (all from Thermo Fisher Scientific). The murine GL-261 brain tumor cell line (syngenic to C57BL/6), was stably transfected with pGI3-ctrl and pGK- Puro (Promega) and selected with puromycin (Sigma-Aldrich) to generate luciferase-stable GL-261 cells.

### Surgical procedures

For glioma inoculation, 6-10 week old mice were anesthetized using a mixture of fentanyl (Helvepharm AG), midazolam (Roche Pharma AG) and medetomidin (Orion Pharma AG). GL261 cells were injected intracranially (i. c.) in the right hemisphere using a stereotactic robot (Neurostar). Briefly, a blunt-ended syringe (Hamilton; 75N, 26s/2"/2, 5 µl) was placed 1.5 mm lateral and 1 mm frontal of bregma. The needle was lowered into the burr hole to a depth of 4 mm below the dura surface and retracted 1 mm to form a small reservoir. Injection was performed in a volume of 2 µl at 1 µl/min. After leaving the needle in place for 2 min, it was retracted at 1 mm/min. The burr hole was closed with bone wax (Aesculap, Braun) and the scalp wound was sealed with tissue glue (Indermil, Henkel). Anesthesia was interrupted using a mixture of flumazenil (Labatec Pharma AG) and Buprenorphine (Indivior Schweiz AG), followed by injection of atipamezol 20 minutes later (Janssen). Carprofen (Pfizer AG) was used for perioperative analgesia.

After 7 to 14 days osmotic pumps (model 2004, 0.25 µl/h; Alzet) were filled with murine IL-12Fc (12.5 µg/kg/24 h) or PBS alone and primed at 37°C in PBS. Mice were anaesthetized as above and the previous burr hole of the glioma injection was located, the bone wax and periosteal bone was removed, and the infusion cannula was lowered through the burr hole 3 mm into the putative center of the tumor. Serum samples were collected every two days by blood sampling from the tail vein, starting from day -1 of the pump implantation using Vacutainer tubes and following manufacturer's instructions (Becton, Dickinson and Company). For the comparison of IL-12 and IL-12Fc WT serum to brain concentration ratio after bolus injection, mice were anesthetized and intracranially injected in the right hemisphere using a stereotactic robot (Neurostar) as described above for tumor cell injection. Mice received 100 ng of recombinant human IL-12 (Prospec) or equivalent amount of IL-12Fc (69 ng/mouse). Dosage was calculated based on the HEK-Blue IL-12 bioactivity assay. Animals were sacrificed 24 hours later by controlled CO₂ asphyxiation. Blood samples were collected by cardiac puncture and mice were perfused with 20 ml of ice cold PBS. Serum was isolated as described above and brain tissue was snap-frozen in liquid nitrogen.

For the comparison of IL-12 WT and IL-12Fc NHQ serum to brain concentration ratio after bolus injection, mice were anesthetized and intracranially injected in the right hemisphere using a stereotactic robot (Neurostar) as described above for tumor cell injection. Mice received 1 µg of human IL-12Fc WT or IL-12Fc NHQ. Animals were sacrificed 24 hours later by controlled CO₂ asphyxiation. Blood samples were collected by cardiac puncture and mice were perfused with 20 ml of ice cold PBS. Serum was isolated as described above and brain tissue was snap-frozen in liquid nitrogen.

For the convection enhanced delivery (CED) of protein into the brain, mice were anesthetized and intracranially injected in the right hemisphere using a stereotactic robot (Neurostar) and catheters made using a 27 G blunt-end needle with a 1 mm step at the tip made of fused silica with internal diameter of 0.1 mm and wall thickness of 0.0325 mm. Briefly, a burr hole was made at position 1 mm anteroposterior and 2 mm mediolateral of bregma. The catheter was lowered into the burr hole to a depth of 3.5 mm below the dura surface. Injection was performed in a volume of 5 µl at 0.2 µl/min, then 2 µl at 0.5 µl/min and 2 µl at 0.8 µl/min. After leaving the needle in place for 2 min, it was retracted at 1 mm/min. Mice received 1µg of recombinant human IL-12Fc WT, IAQ, AAA or NHQ. Animals were sacrificed 6 hours later by controlled CO₂ asphyxiation. Ipsilateral brain hemispheres were snap-frozen in liquid nitrogen.

### FcRn ELISA

IL-12Fc variants or a recombinant human IgG4 anti-GFP antibody (clone 515, AbD Serotec), which served as a control, were coated on a micro-well plate (Greiner Bio-One). Histidine-coupled FcRn (R&D Systems) was incubated at increasing concentration in ELISA diluent (Mabtech) at pH=6.0. FcRn was detected by a biotinylated anti-His-antibody (clone 13/45/31-2, Dianova), streptavidin-coupled horseradish peroxidase (Mabtech) and a colorimetric substrate (Chromogen-TMB, Thermo Fisher Scientific). Optical density at a wavelength of 450 nm was measured using a spectrophotometer (Molecular Devices).

### Beads-based cytokine array

Serum levels of mIL-12 and mIFN-γ were measured using Legendplex Mouse Inflammation Panel (Biolegend) following manufacturer's instructions. Samples were acquired using LSRII Fortessa (Becton, Dickinson and Company).

### HEK-Blue IL-12 bioactivity assay

HEK-Blue IL-12 cells (InvivoGen) were plated on a flat bottom 96 well plates (Corning) at a density of 50 000 cells /well in medium containing normocin (InvivoGen). Cells were incubated with increasing amounts of IL-12, IL-12Fc WT or variants designed for reduced FcRn affinity for 17 hours. Culture medium was collected and incubated for 2 hours in presence of Quanti-Blue detection reagent (InvivoGen). Absorbance was measured at 640 nm using a table top spectrophotometer (Molecular Devices).

### IL-12 detection in the brain, plasma and serum after injection into the brain and calculation of the serum or plasma to brain concentration ratio

Samples were diluted in PBS containing 0.05% Tween-20 and 0.1% BSA and IL-12 levels were assessed by ELISA for hIL-12p70 (Mabtech). To calculate serum or plasma to brain concentration ratio, the concentration of IL-12 in serum or plasma was described in pg/ml, whereas concentration in brain was calculated by dividing the total amount of IL-12 extracted from the brain corrected for the efficacy of protein extraction by the weight of the hemisphere (pg/mg of brain tissue).

### IFN-γ production by lymphocytes stimulated with IL-12Fc

Human peripheral blood mononuclear cells (PBMCs) were stimulated for 24 h with increasing concentrations of IL-12, IL-12Fc or IL-12Fc variants with reduced FcRn affinity in presence of 100 ng/ml anti-CD3 antibody. IFN-γ levels in supernatant were measured by ELISA following manufacturer's instructions (Mabtech).

### Brain protein isolation

After euthanasia and careful removal of the skullcap the brain was isolated. Cerebellum and olfactory bulbs were removed, the hemispheres are separated along the midline and the injected (ipsilateral) hemisphere was snap frozen in liquid nitrogen. Brain lysates were prepared by homogenization in ice cold lysis buffer (Cell signaling) containing Halt protease inhibitor cocktail (Thermo Fisher Scientific). 0.1 ml of lysis buffer was added per 10 mg of brain tissue. Brain tissue was minced using scissors, then passed through a 20 G needle and finally sonicated for 20 seconds. Samples were spun down for 10 minutes at 15000 g at 4°C and supernatants were transferred to fresh tubes. Protein concentration was measured using Pierce BCA assay kit (Thermo Fisher Scientific) and this data was used to correct for the protein extraction efficiency within each experiment.

### Example 2: Intracranial injection of human IL-12 has higher systemic leakage than hIL-12Fc

IL-12Fc for the local treatment of brain tumors is very promising. However, for use in clinical trials a human version of IL-12Fc is required that needs to show similar properties. To obtain a human analogue to murine IL-12IgG3 the inventors fused single chain human IL-12 to the crystallisable fragment (Fc) of human immunoglobulin G4 (hlgG4) (Fig. 1A). Similar to mIgG3, hIgG4 does not support antibody dependent cell-mediated cytotoxicity (ADCC) and does not activate the complement system. To test for leakage and stability of human IL-12Fc (hIL-12Fc) vs recombinant human IL-12 (rhIL-12), the inventors injected a single bolus into the striatum of transgenic mice that express the human FcRn on a murine FcRn-deficient background (FcRntg) (Postow et al., 2015, N Engl J Med 372:2006-2017; Kamran et al., 2016, Expert Opin Biol Ther 16:1245-1264). After 24 hours, the inventors analysed the concentration of human IL-12 in the lysate of the ipsilateral hemisphere and in the serum to learn more about the stability and retention at the site of injection (residual concentration) and the rate of leakage into the bloodstream (Fig. 1B). For each mouse, the inventors calculated the ratios of concentrations in the serum vs the concentration at the injection site as an estimate for tissue retention. Comparing serum levels to local concentration at the injection site, hIL- 12Fc showed superior tissue retention over rhIL-12, as the inventors observed considerably lower ratios (Fig. 1C). For local GB treatment, a human IL-12Fc fusion cytokine seems to be a superior compound compared to its natural counterpart due to its higher tissue retention, stability and solubility.

### Example 3: FcRn binding leads to systemic accumulation of IL-12Fc

The neonatal Fc receptor (FcRn)-based endosomal recycling system in endothelial cells and red pulp macrophages prevents rapid degradation and clearance of IgG. After pinocytosis, facilitated by the acidic pH of endosomes, FcRn binds IgG and recycles it to the cell surface, where neutral pH induces its release. When injected locally, IL-12Fc can leak from the brain in an FcRn-mediated fashion due to its Fc tag. Leakage from the brain leads to IL-12Fc serum accumulation and may ultimately reach toxic levels. To test whether FcRn-based recycling indeed promotes accumulation of hIL-12Fc in the serum, the inventors utilized transgenic mice that express the human FcRn on a murine FcRn-deficient background (FcRntg). Since human FcRn has a weak affinity to murine IgG, but promotes normal albumin recycling, only murine IgG recycling is impaired in this mouse model. Murine IL-12Fc should therefore bind considerably less to FcRn in these FcRn humanized mice. Thus, the inventors compared serum mIL-12 levels of glioma-bearing wild type (wt) and FcRntg mice that were being treated with local murine IL-12Fc via osmotic minipumps. Indeed, after a week the inventors observed an increase in IL-12 levels in wt mice that was less pronounced in FcRntg mice (Fig. 2A) followed by an increase in IFN-γ levels (Fig. 2D). The inventors even observed elevated levels of IL-12 in the sera of some mice as early as day 1 after pump implantation (Fig. 2B). Consequently, this led to noticeable increase of IFN-γ serum levels in wt mice, which was not the case in the FcRntg cohort (Fig. 2C). Similar to murine IL-12Fc, human IL-12Fc will most likely also leak and accumulate, posing a threat for systemic side effects. Moreover, IFN-γ is one of the main mediators of the IL-12 related side effects (Leonard et al., 1997, Blood 90:2541-2548) and its persistent systemic presence can be toxic (Weiss et al., 2007, Expert Opin Biol Ther 7:1705-1721). Taken together, the inventors conclude that the leakage of even minute amounts of IL-12Fc from the treatment site is sufficient to trigger detectable serum IFN-γ levels.

### Example 4: Generation of human IL-12Fc variants designed for improved tissue retention

The observation that reduced FcRn binding potentially abrogates export from the brain and leads to dramatically reduced recycling upon leakage out of the brain can be exploited to increase the safety margin of hIL-12Fc. The inventors therefore set out to reduce the binding of the Fc portion of hIL-12Fc to human FcRn. By increasing the positive charge at the FcRn binding interface of the Fc part this interaction at acidic pH - and hence the recycling - can be abrogated, which was shown to decrease serum half-life of immunoglobulins. The inventors introduced a number of mutations into hIL-12Fc (Table 1), at the FcRn binding site of hIL-12Fc with the aim of decreasing its serum half-life in case of leakage.

The inventors have generated three IL-12Fc variants with mutations analogous to the previously published antibodies with reduced FcRn affinity called IAQ, AHH and AAA. Furthermore, the inventors substituted the isoleucine at position 253 not to alanine, which represents a simple shortening of the sidechain, but changed it to asparagine instead (I253N). Asparagine is a polar amino acid, whose sidechain has a similar length as isoleucine. The inventors have also modified histidine at position 310 to alanine and at position 435 to glutamine, alanine or glutamic acid.

All the variants were expressed in human embryonic kidney 293T cell (HEK293T) cultures. Expression levels for all the variants were similar.

### Example 5: Human IL-12Fc variants have similar protein stability

First, the inventors have validated if the changes introduced to the Fc influence the overall protein stability. To this end the inventors have measured the denaturation temperature for each of the variant in a Thermal Shift Assay performed in PBS as well as in artificial cerebrospinal fluid (aCSF). The denaturation temperature for all the variants oscillated around 60°C (Fig. 3 A). Measurements performed in aCSF confirmed that all the variants have similar stability, although the overall denaturation temperature was lower - approximately 57°C (Fig. 3 B).

### Example 6: Human IL-12Fc variants sustain their biological activity

Even though the inventors aimed to reduce the binding of hIL-12Fc to FcRn the inventors could not exclude that those changes had influences on the IL-12 biological activity. This was tested first using a HEK cell line stably transfected with IL-12 signaling components and a downstream enzyme catalyzing a colorimetric reaction. Only the NAQ variant showed approximately 2x reduced activity compared to IL-12Fc, whereas all the others had an EC50 in the range of IL-12Fc WT (Fig. 4 A). Importantly, IL-12Fc had comparable activity *in vitro* as rIL-12.

To further validate activity of IL-12Fc variants, the inventors performed activation of peripheral blood mononuclear cells (PBMCs) with three different hIL-12Fc variants, namely IAQ, AHQ and NHQ, followed by analysis of STAT-4 phosphorylation (Fig. 4 B). More importantly, this STAT-4 phosphorylation translated into robust production of IFN-γ (Fig. 4 C) 24h later, indicating that all of the variants retained the activity of rhIL-12.

### Example 7: Human IL-12Fc variants differ in binding to protein G

Protein A and G affinity chromatography are among the standard methods used for purification of recombinant antibodies and Fc-fusion proteins. Modifying the interface between Fc and FcRn is known to abrogate Protein A binding, an observation that the inventors also confirmed with IL-12Fc variants. In order to confirm feasibility of production in a scaled-up process the inventors decided to validate the possibility of purifying IL-12Fc variants via Protein G affinity columns. The majority of the inventors' variants retained affinity to Protein G, but to the inventors' surprise, all the variants containing both I253N together with the H310A mutations were not suitable for protein G purification (Table 2). This effect was independent of additional mutations on position 435. For further studies the inventors have focused their attention on the variants with retained Protein G affinity.

### Example 8: Human IL-12Fc variants have reduced FcRn affinity

In order to validate the affinity of the IL-12Fc variants to FcRn the inventors used surface plasmon resonance (SPR), a label-free method to characterize protein-protein interactions. The inventors immobilized human FcRn and measured the binding of IL-12Fc variants at lysosomal pH ranges (pH = 6.0) in various concentrations (43). As shown on Figure 5 A, the majority of the modified IL-12Fc variants have lowered affinity to human FcRn, with the NHQ variant showing the strongest reduction (approximately 8x lower). The inventors used a commercially available human monoclonal anti-GFP IgG4 antibody as a control. Furthermore, the inventors corroborated these data with ELISA data for the NHQ construct using IL-12Fc WT, anti-GFP IgG4 and the published variant IAQ as references. Both IAQ and NHQ showed reduced binding, with the NHQ having the lowest affinity (Fig. 5 B). The inventors thus conclude that the NHQ combination of substitutions seemed to reduce the binding to FcRn most dramatically. This is in contrast to results from Kenanova and colleagues (Kenanova et al., 2005, Cancer Research 65:622-631) who suggested that the combined mutations H310A and H435Q are responsible for the strongest reduction of binding to FcRn at low pH.

### Example 9: Introduction of NHQ mutation reduces the systemic exposure to locally delivered hIL-12Fc

The inventors hypothesized that reducing the FcRn affinity will increase the retention of hIL-12Fc in the CNS and at the same time prohibit its systemic accumulation. This was addressed in a similar fashion to the comparison of hIL-12Fc WT and recombinant human IL-12 (Fig. 1 B). FcRn^{tg} mice were injected with a single dose of 1 µg of IL-12Fc WT or the NHQ variant and IL-12 was measured by ELISA in the ipsilateral brain hemisphere and in serum. Mice injected with the NHQ variant showed lowered serum-to-brain ratios compared to mice injected with hIL-12Fc WT (Fig. 6 A). The inventors postulate that this can be an effect of both increased retention in the CNS as well as attenuated systemic accumulation due to the FcRn-mediated recycling.

Furthermore, using CED instead of bolus injection, the inventors have compared the concentrations of hIL-12Fc WT, IAQ, AAA and NHQ in plasma and the injected hemisphere 24 h after CED and observed that the NHQ variant exhibits the most significantly reduced plasma to brain ratio (Fig. 6 B) also in optimized delivery settings compared to bolus injection. Such increased CNS retention merged with lowered systemic exposure could potentially improve the safety profile of local IL-12Fc therapy.

### Example 10: IL-12Fc variant NHQ has higher brain tissue retention than other low affinity variants

Finally, the inventors measured the tissue retention after intracranial delivery of protein. To this end, the inventors injected 1 µg of unmodified IL-12Fc WT, the two previously published variants with reduced FcRn affinity, namely IAQ and AAA as well as NHQ, a variant with the lowest FcRn affinity according to the inventors' measurements (Fig. 5 A). In order to ensure maximal perfusion of the brain hemisphere, instead of a bolus injection of the protein solution, the inventors have used a CED protocol with a step catheter and a ramp-up injection regimen. To study the effect of different modifications at the FcRn binding interface in a most physiological setting, the inventors employed FcRn^{tg} mice. As discussed earlier, FcRn is important for both the export from CNS and accumulation of Fc-containing molecules in the serum. In an attempt to decouple the two effects and focus solely on preventing the transport from CNS, the inventors have measured the amount of protein left in the brain 6 hours after the CED. Mice were euthanized, perfused with PBS, total protein in the ipsilateral hemisphere was isolated and hIL-12 was measured by ELISA. As shown on Fig. 7, IL-12Fc NHQ has superior tissue retention compared to IL-12Fc WT. Importantly, it was also better than IAQ and AAA, the two other variants with reduced FcRn affinity. Surprisingly, IAQ and AAA were not significantly different than IL-12Fc WT.

### Discussion

The finding that hIL-12Fc is functional, has higher tissue retention than rhIL-12 and that abrogation of systemic recycling can increase the safety margin in case of leakage has potentially wide implications for the local administration of any Fc containing molecule. It is tempting to speculate that these modifications could enable safe and efficacious local delivery of any antibody or Fc-fusion molecule for the local treatment of neurologic diseases.

Administration of therapeutics into the CNS via the systemic route (either per os or i.v.) is challenging mainly because of the BBB and - compared to the rest of the body - only a small selection of today's therapeutics actually reaches the brain. Unfortunately, antibodies and Fc containing biologics, particularly Fc-fusion proteins, do not readily cross the BBB and in addition are actively exported. Enabling transport of antibodies over the BBB into the brain parenchyma is being extensively studied, e.g. by exploiting receptor mediated transcytosis of transferrin. Cytokines have a short half-life in circulation and bear a high risk of adverse effects, which narrows their therapeutic opportunity window. Cytokines can be linked to antibodies homing to tumors, where they will accumulate, particularly NHS-IL-12. Even after subcutaneous dosing, these antibodies induce an IFN-γ response as they travel to the tumor via the bloodstream. Initially, systemic delivery of IL-12 was assessed for treatment of non-brain cancers. However, these clinical trials had to be prematurely terminated, since - at effective doses - intravenous application led to serious adverse events, including deaths. One of the main reasons seems to have been the induction of IFN-γ by IL-12.

The serum half-life and solubility of protein therapeutics can be improved by direct fusion of the therapeutic moiety with the crystallizable fragment (Fc) of antibodies. For direct local application in anatomically distinct locations this can lead also to less desirable effects. One of these can be FcRn-mediated export of Fc-containing molecules from immune privileged anatomical sites, particularly the brain and their serum accumulation analogous to IgG recycling.

The inventors have observed that local administration of an IL-12Fc fusion cytokine into the brain triggers FcRn dependent export of IL-12Fc through the BBB into the circulation. IL-12Fc accumulates in the blood and triggers potentially dangerous IFN-γ production.

The inventors found that IL-12Fc with reduced FcRn affinity is functional and has higher tissue retention than recombinant IL-12, as well as unmodified IL-12Fc. When compared in a brain tissue retention experiment, the NHQ mutant was the only one with improved retention over IL-12Fc WT. Surprisingly, IAQ and AAA, two variants reported to have dramatically reduced FcRn binding were not different than unmodified IL-12Fc, suggesting that in order to obtain biological difference, the FcRn affinity must be reduced over a given threshold, that only the NHQ modification reaches. Alternatively, it cannot be ruled out that the NHQ mutations introduce other features that improve tissue retention in an FcRn-independent way.

This translates into an improved safety profile and broadens the therapeutic window for the IL-12Fc therapy of brain tumors. Moreover, the inventors' findings can be translated to any Fc containing therapeutics, primarily therapeutic antibodies where there is a strong rationale for local intracranial administration. Such an application route would be preferred due to weak efficacy when given systemically, potentially an effect of poor crossing through the BBB, or because the desired therapeutic effect should be contained locally. Local therapy with biologics optimized for such delivery should preclude the systemic toxicity and thus improve the safety profile of the drug.

**Table 1. List of the mutations introduced to the Fc part of IL-12Fc. Amino acid positions numbered according to EU numbering system (Edelman et al. Proceedings of the National Academy of Sciences of the United States of America (1969) 63(1):78-85).**

| Name | SEQ ID NO. | Position 253 | Position 310 | Position 435 |
|---|---|---|---|---|
| Fc WT | SEQ ID NO. 001 | I | H | H |
| IAQ | SEQ ID NO. 002 | I | A | Q |
| AHQ | SEQ ID NO. 003 | A | H | Q |
| NHQ | SEQ ID NO. 004 | N | H | Q |
| AAQ | SEQ ID NO. 005 | A | A | Q |
| NAQ | SEQ ID NO. 006 | N | A | Q |
| AHH | SEQ ID NO. 007 | A | H | H |
| NHH | SEQ ID NO. 008 | N | H | H |
| AAH | SEQ ID NO. 009 | A | A | H |
| NAH | SEQ ID NO. 010 | N | A | H |
| NAA | SEQ ID NO. 011 | N | A | A |
| NAE | SEQ ID NO. 012 | N | A | E |
| AAA | SEQ ID NO. 013 | A | A | A |
| AAE | SEQ ID NO. 014 | A | A | E |

**Table 2. List of IL-12Fc variants and their ability to bind to Protein G.**

| Name | SEQ ID NO. | Retained affinity to the Protein G chromatography bead: |
|---|---|---|
| Fc wt | SEQ ID NO. 001 | + |
| IAQ | SEQ ID NO. 002 | + |
| AHQ | SEQ ID NO. 003 | + |
| NHQ | SEQ ID NO. 004 | + |
| AAQ | SEQ ID NO. 005 | + |
| NAQ | SEQ ID NO. 006 | - |
| AHH | SEQ ID NO. 007 | + |
| NHH | SEQ ID NO. 008 | + |
| AAH | SEQ ID NO. 009 | + |
| NAH | SEQ ID NO. 010 | - |
| NAA | SEQ ID NO. 011 | - |
| NAE | SEQ ID NO. 012 | - |
| AAA | SEQ ID NO. 013 | + |
| AAE | SEQ ID NO. 014 | + |

**Table 3. List of sequences of molecules, which combination makes a bispecific antibody or antibody-like molecule binding to human or mouse IL-12Fc- and human or mouse PD-L1.**

| Name | SEQ ID NO. |
|---|---|
| mIL-12Fc-IgG4 NHQ Hole | SEQ ID NO. 015 |
| hIL-12Fc-IgG4 NHQ Hole | SEQ ID NO. 016 |
| mIL-12Fc-IgG1 short NHQ Hole | SEQ ID NO. 017 |
| hIL-12Fc-IgG1 short NHQ Hole | SEQ ID NO. 018 |
| mIL-12Fc-IgG1 long NHQ Hole | SEQ ID NO. 019 |
| hIL-12Fc-IgG1 long NHQ Hole | SEQ ID NO. 020 |
| anti-PD-L1 scFv-IgG4 NHQ Knob | SEQ ID NO. 021 |
| anti-PD-L1 scFv-IgG1 NHQ Knob | SEQ ID NO. 022 |
| anti-PD-L1 HC-IgG1 NHQ Knob | SEQ ID NO. 023 |
| anti-PD-L1 LC-IgG1 | SEQ ID NO. 024 |

A combined bispecific molecule may consist of molecules described as sequence SEQ ID NO 15 with SEQ ID NO 21, SEQ ID NO 16 with SEQ ID NO 21, SEQ ID NO 17 with SEQ ID NO 22, SEQ ID NO 18 with SEQ ID NO 22, SEQ ID NO 17 with SEQ ID NO 23 and SEQ ID NO 24, SEQ ID NO 18 with SEQ ID NO 23 and 24, SEQ ID NO 19 with SEQ ID NO 22, SEQ ID NO 20 with SEQ ID NO 22, SEQ ID NO 19 with SEQ ID NO 23 and SEQ ID NO 24, SEQ ID NO 20 with SEQ ID NO 23 and SEQ ID NO 24.

## Claims

1. A fusion polypeptide comprising IL-12 and a crystallizable fragment (Fc) region of IgG, for use in prevention or treatment of a disease affecting the central nervous system (CNS), particularly a primary and secondary brain cancer, wherein
said Fc region bears a modification resulting in reduced affinity to the neonatal Fc receptor (FcRn)and
said polypeptide is administered to the brain.

2. The polypeptide for use in prevention or treatment of a disease affecting the central nervous system according to claim 1, wherein the serum or plasma to brain concentration ratio of said polypeptide is below a predetermined threshold is selected from
a. at most 2/3 of the serum or plasma to brain concentration ratio of the same polypeptide comprising a non-modified Fc region, or
b. at most 1/8 of the serum or plasma to brain concentration ratio of the same polypeptide neither comprising an Fc region nor peptide linkers,
measurable 24 h after intracranial bolus injection into the striatum of FcRn^{tg} mice.

3. The polypeptide for use in prevention or treatment of a disease affecting the central nervous system according to any one of the above claims, wherein said reduced affinity of said polypeptide to FcRn is **characterized by** a dissociation constant (K_{D}) selected from
a. a K_{D} that is at least 2x, particularly at least 3x, more particularly at least 4x, even more particularly at least 5x increased compared to a K_{D} characterizing binding of FcRn to the same polypeptide comprising a non-modified Fc region, and
b. a K_{D} that is at least 1.5x, particularly at least 2x increased compared to a K_{D} characterizing binding of FcRn to the same polypeptide comprising a differently modified Fc region, namely one mutant selected from IAQ and AAA

4. The polypeptide for use in treatment or prevention of a disease affecting the central nervous system according to any one of the above claims, wherein said intracranial delivery is effected by a method selected from
a. single, intermittent or continous local infusion, including convection enhanced delivery (CED),
b. intrathekal or intracerebroventricular administration,
c. in situ production of said polypeptide,
d. release from implanted slow release formulations,
e. molecular transport into the CNS,
f. cellular transport into the CNS, or
g. transport to the CNS after intranasal application.

5. The polypeptide for use in treatment or prevention of a disease affecting the central nervous system according to any one of the above claims, wherein said disease affecting the central nervous system is a malignant disease, particularly a glioma, more particularly a high grade glioma (HGG).

6. The polypeptide for use in prevention or treatment of a disease affecting the central nervous system according to any one of the above claims, wherein said Fc region is a human Fc region or a chimeric Fc region comprising a human amino acid sequence and bears a mutation at position 253, in particular I253A or I253N, more particularly I253N.

7. The polypeptide for use in prevention or treatment of a disease affecting the central nervous system according to any one of the above claims, wherein said Fc region is or comprises a sequence **characterized by** SEQ ID NO 002 (IAQ), SEQ ID NO 003 (AHQ), SEQ ID NO 004 (NHQ), SEQ ID NO 005 (AAQ), SEQ ID NO 006 (NAQ), SEQ ID NO 007 (AHH), SEQ ID NO 008 (NHH), SEQ ID NO 009 (AAH), SEQ ID NO 010 (NAH), SEQ ID NO 011 (NAA), SEQ ID NO 012 (NAE), SEQ ID NO 013 (AAA) or SEQ ID NO 014 (AAE).

8. The polypeptide for use in prevention or treatment of a disease affecting the central nervous system according to any one of the above claims, wherein said Fc region is or comprises a sequence **characterized by** SEQ ID NO 004 (NHQ), SEQ ID NO 005 (AAQ), SEQ ID NO 006 (NAQ), SEQ ID NO 012 (NAE) or SEQ ID NO 014 (AAE), particularly wherein said Fc region is or comprises a sequence **characterized by** SEQ ID NO 004 (NHQ).

9. A polypeptide comprising a Fc region of IgG, wherein said Fc region bears a modification resulting in reduced affinity to the neonatal Fc receptor (FcRn), wherein said Fc region is or comprises a sequence **characterized by** SEQ ID NO 002 (IAQ), SEQ ID NO 003 (AHQ), SEQ ID NO 004 (NHQ), SEQ ID NO 005 (AAQ), SEQ ID NO 006 (NAQ), SEQ ID NO 007 (AHH), SEQ ID NO 008 (NHH), SEQ ID NO 009 (AAH), SEQ ID NO 010 (NAH), SEQ ID NO 011 (NAA), SEQ ID NO 012 (NAE), SEQ ID NO 013 (AAA) or SEQ ID NO 014 (AAE).

10. The polypeptide according to claim 9, wherein said Fc region is or comprises a sequence **characterized by** SEQ ID NO 004 (NHQ), SEQ ID NO 005 (AAQ), SEQ ID NO 006 (NAQ), SEQ ID NO 012 (NAE) or SEQ ID NO 014 (AAE), particularly wherein said Fc region is or comprises a sequence **characterized by** SEQ ID NO 004 (NHQ).

11. The polypeptide according to claim 9 or 10, wherein said polypeptide is selected from
a. a fusion protein comprising
i. an effector polypeptide and
ii. said Fc region; or
b. an antibody or antibody-like molecule comprising said Fc region.

12. The polypeptide according to claim 11, wherein said effector polypeptide is selected from hIL-12, IL-10, IL-2, IL-7, IFNα, IFNβ, IFNγ, IL-15, TNFα, CTLA-4, TGFβ, TGFβRII, GDNF, hIL-35, CD95, IL-1RA, IL-4, IL-13, SIRPα, G-CSF, GM-CFS, OX40L, CD80, CD86, GITRL, 4-1BBL, EphrinA1, EphrinB2, and EphrinB5, BDNF, C9orf72, NRTN, ARTN, PSPN, CNTF, TRAIL, IFNα, IFNβ, IL-4, IL-3, IL-1, IL-5, IL-8, IL-18, IL-21, CCL5, CCL21, CCL10, CCL16, CX3CL1, and CXCL16, in particular said effector polypeptide is hIL-12.

13. The polypeptide according to claim 11, wherein said antibody or antibody-like molecule is selected from an antibody or antibody-like molecule specifically binding to a molecule selected from PD-L1, TNFα, Histone, IFNγ, CXCL10, CTLA4, PD-1, OX40, CD3, CD25, CD28, TREM2, IL-6, CX3CR1, Nogo-A, CD25, CD27, IL-12, IL-12Rb1, IL-23, CD47, TGFβ, EGFR, EGFRvIII, Her2, PDGFR, TGFR, FGFR, IL-4, IL-4RA, TfR, LfR, IR, LDL-R, LRP-1, CD133, CD111, VEGFR, VEGF-A, Ang-2, IL-10, IL-10R, IL-13Rα2, α-synuclein, CSF1R, GITR, TIM-3, LAG-3, TIGIT, BTLA, VISTA, CD96, 4-1BB, CCL2, IL-1 or IL-1R, EphA2, EphA3, EphB2, EphB3, and EphB4, LINGO-1, L1CAM, NCAM, SOD-1, SIGMAR-1, SIGMAR-2, TDP-43, Aβ, Tau, IFNα, IFNβ, TRPM4, ASIC1, VGCCs, CB₁,TTR, HTT, JCV, and C9orf72, in particular said antibody or antibody-like molecule is an antibody specifically binding to a molecule selected from PD-L1, OX40, CD47, and Nogo-A.

14. The polypeptide according any one of claims 9 to 13 for use in treatment of a disease selected from brain cancer, stroke, dementia, Parkinson's disease, Alzheimer's disease, multiple sclerosis, epilepsy, and traumatic CNS injury.

15. A nucleic acid encoding the polypeptide according to any one of claims 9 to 14, or a viral vector comprising the nucleic acid according to claim 15.


SEQUENCE LISTING

<110>  University of Zurich

<120>  Fc-modified biologicals for local CNS delivery

<130>  uz313ep

<160>  24

<170>  PatentIn version 3.5

<210>  1
<211>  235
<212>  PRT
<213>  Homo sapiens

<400>  1

Lys Val Asp Lys Arg Val Glu Ser Lys Tyr Gly Pro Pro Cys Pro Ser
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Ile Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu His Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn His Tyr
    210                 215                 220

Thr Gln Lys Ser Leu Ser Leu Ser Leu Gly Lys
225                 230                 235

<210> 2
<211> 235
<212> PRT
<213> Homo sapiens

<400> 2

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Ile Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu Ala Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn Gln Tyr
    210                 215                 220

Thr Gln Lys Ser Leu Ser Leu Ser Leu Gly Lys
225                 230                 235

<210>  3
<211>  235
<212>  PRT
<213>  Homo sapiens

<400>  3

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Ala Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu His Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn Gln Tyr
    210                 215                 220

Thr Gln Lys Ser Leu Ser Leu Ser Leu Gly Lys
225                 230                 235

<210>  4
<211>  235
<212>  PRT
<213>  Homo sapiens

<400>  4

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Asn Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu His Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn Gln Tyr
    210                 215                 220

Thr Gln Lys Ser Leu Ser Leu Ser Leu Gly Lys
225                 230                 235

<210>  5
<211>  235
<212>  PRT
<213>  Homo sapiens

<400>  5

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Ala Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu Ala Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn Gln Tyr
    210                 215                 220

Thr Gln Lys Ser Leu Ser Leu Ser Leu Gly Lys
225                 230                 235

<210>  6
<211>  235
<212>  PRT
<213>  Homo sapiens

<400>  6

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Asn Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu Ala Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn Gln Tyr
    210                 215                 220

Thr Gln Lys Ser Leu Ser Leu Ser Leu Gly Lys
225                 230                 235

<210> 7
<211> 235
<212> PRT
<213> Homo sapiens

<400> 7

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Ala Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu His Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn His Tyr
    210                 215                 220

Thr Gln Lys Ser Leu Ser Leu Ser Leu Gly Lys
225                 230                 235

<210>  8
<211>  235
<212>  PRT
<213>  Homo sapiens

<400>  8

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Asn Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu His Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn His Tyr
    210                 215                 220

Thr Gln Lys Ser Leu Ser Leu Ser Leu Gly Lys
225                 230                 235

<210>  9
<211>  235
<212>  PRT
<213>  Homo sapiens

<400>  9

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Ala Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu Ala Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn His Tyr
    210                 215                 220

Thr Gln Lys Ser Leu Ser Leu Ser Leu Gly Lys
225                 230                 235

<210>  10
<211>  235
<212>  PRT
<213>  Homo sapiens

<400>  10

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Asn Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu Ala Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn His Tyr
    210                 215                 220

Thr Gln Lys Ser Leu Ser Leu Ser Leu Gly Lys
225                 230                 235


<210>  11
<211>  235
<212>  PRT
<213>  Homo sapiens

<400>  11

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Asn Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu Ala Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn Ala Tyr
    210                 215                 220

Thr Gln Lys Ser Leu Ser Leu Ser Leu Gly Lys
225                 230                 235

<210> 12
<211> 235
<212> PRT
<213> Homo sapiens

<400> 12

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Asn Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu Ala Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn Glu Tyr
    210                 215                 220

Thr Gln Lys Ser Leu Ser Leu Ser Leu Gly Lys
225                 230                 235

<210>  13
<211>  235
<212>  PRT

<213>  Homo sapiens

<400>  13

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Ala Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu Ala Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn Ala Tyr
    210                 215                 220

Thr Gln Lys Ser Leu Ser Leu Ser Leu Gly Lys
225                 230                 235

<210>  14
<211>  235
<212>  PRT
<213>  Homo sapiens

<400>  14

Lys Val Asp Lys Arg Val Glu Ser Lys Tyr Gly Pro Pro Cys Pro Pro
1               5                   10                  15

Cys Pro Ala Pro Glu Phe Leu Gly Gly Pro Ser Val Phe Leu Phe Pro
            20                  25                  30

Pro Lys Pro Lys Asp Thr Leu Met Ala Ser Arg Thr Pro Glu Val Thr
        35                  40                  45

Cys Val Val Val Asp Val Ser Gln Glu Asp Pro Glu Val Gln Phe Asn
    50                  55                  60

Trp Tyr Val Asp Gly Val Glu Val His Asn Ala Lys Thr Lys Pro Arg
65                  70                  75                  80

Glu Glu Gln Phe Asn Ser Thr Tyr Arg Val Val Ser Val Leu Thr Val
                85                  90                  95

Leu Ala Gln Asp Trp Leu Asn Gly Lys Glu Tyr Lys Cys Lys Val Ser
            100                 105                 110

Asn Lys Gly Leu Pro Ser Ser Ile Glu Lys Thr Ile Ser Lys Ala Lys
        115                 120                 125

Gly Gln Pro Arg Glu Pro Gln Val Tyr Thr Leu Pro Pro Ser Pro Glu
    130                 135                 140

Glu Met Thr Lys Asn Gln Val Ser Leu Thr Cys Leu Val Lys Gly Phe
145                 150                 155                 160

Tyr Pro Ser Asp Ile Ala Val Glu Trp Glu Ser Asn Gly Gln Pro Glu
                165                 170                 175

Asn Asn Tyr Lys Thr Thr Pro Pro Val Leu Asp Ser Asp Gly Ser Phe
            180                 185                 190

Phe Leu Tyr Ser Arg Leu Thr Val Asp Lys Ser Arg Trp Gln Glu Gly
        195                 200                 205

Asn Val Phe Ser Cys Ser Val Met His Glu Ala Leu His Asn Glu Tyr
    210                 215                 220

Thr Gln Lys Ser Leu Ser Leu Ser Leu Gly Lys
225                 230                 235

<210>  15
<211>  778
<212>  PRT
<213>  Mus musculus

<400>  15

Met Cys Pro Gln Lys Leu Thr Ile Ser Trp Phe Ala Ile Val Leu Leu
1               5                   10                  15

Val Ser Pro Leu Met Ala Met Trp Glu Leu Glu Lys Asp Val Tyr Val
            20                  25                  30

Val Glu Val Asp Trp Thr Pro Asp Ala Pro Gly Glu Thr Val Asn Leu
        35                  40                  45

Thr Cys Asp Thr Pro Glu Glu Asp Asp Ile Thr Trp Thr Ser Asp Gln
    50                  55                  60

Arg His Gly Val Ile Gly Ser Gly Lys Thr Leu Thr Ile Thr Val Lys
65                  70                  75                  80

Glu Phe Leu Asp Ala Gly Gln Tyr Thr Cys His Lys Gly Gly Glu Thr
                85                  90                  95

Leu Ser His Ser His Leu Leu Leu His Lys Lys Glu Asn Gly Ile Trp
            100                 105                 110

Ser Thr Glu Ile Leu Lys Asn Phe Lys Asn Lys Thr Phe Leu Lys Cys
        115                 120                 125

Glu Ala Pro Asn Tyr Ser Gly Arg Phe Thr Cys Ser Trp Leu Val Gln
    130                 135                 140

Arg Asn Met Asp Leu Lys Phe Asn Ile Lys Ser Ser Ser Ser Ser Pro
145                 150                 155                 160

Asp Ser Arg Ala Val Thr Cys Gly Met Ala Ser Leu Ser Ala Glu Lys
                165                 170                 175

Val Thr Leu Asp Gln Arg Asp Tyr Glu Lys Tyr Ser Val Ser Cys Gln
            180                 185                 190

Glu Asp Val Thr Cys Pro Thr Ala Glu Glu Thr Leu Pro Ile Glu Leu
        195                 200                 205

Ala Leu Glu Ala Arg Gln Gln Asn Lys Tyr Glu Asn Tyr Ser Thr Ser
    210                 215                 220

Phe Phe Ile Arg Asp Ile Ile Lys Pro Asp Pro Pro Lys Asn Leu Gln
225                 230                 235                 240

Met Lys Pro Leu Lys Asn Ser Gln Val Glu Val Ser Trp Glu Tyr Pro
                245                 250                 255

Asp Ser Trp Ser Thr Pro His Ser Tyr Phe Ser Leu Lys Phe Phe Val
            260                 265                 270

Arg Ile Gln Arg Lys Lys Glu Lys Met Lys Glu Thr Glu Glu Gly Cys
        275                 280                 285

Asn Gln Lys Gly Ala Phe Leu Val Glu Lys Thr Ser Thr Glu Val Gln
    290                 295                 300

Cys Lys Gly Gly Asn Val Cys Val Gln Ala Gln Asp Arg Tyr Tyr Asn
305                 310                 315                 320

Ser Ser Cys Ser Lys Trp Ala Cys Val Pro Cys Arg Val Arg Ser Gly
                325                 330                 335

Gly Gly Gly Ser Gly Gly Gly Gly Ser Gly Gly Gly Gly Ser Arg Val
            340                 345                 350

Ile Pro Val Ser Gly Pro Ala Arg Cys Leu Ser Gln Ser Arg Asn Leu
        355                 360                 365

Leu Lys Thr Thr Asp Asp Met Val Lys Thr Ala Arg Glu Lys Leu Lys
    370                 375                 380

His Tyr Ser Cys Thr Ala Glu Asp Ile Asp His Glu Asp Ile Thr Arg
385                 390                 395                 400

Asp Gln Thr Ser Thr Leu Lys Thr Cys Leu Pro Leu Glu Leu His Lys
                405                 410                 415

Asn Glu Ser Cys Leu Ala Thr Arg Glu Thr Ser Ser Thr Thr Arg Gly
            420                 425                 430

Ser Cys Leu Pro Pro Gln Lys Thr Ser Leu Met Met Thr Leu Cys Leu
        435                 440                 445

Gly Ser Ile Tyr Glu Asp Leu Lys Met Tyr Gln Thr Glu Phe Gln Ala
    450                 455                 460

Ile Asn Ala Ala Leu Gln Asn His Asn His Gln Gln Ile Ile Leu Asp
465                 470                 475                 480

Lys Gly Met Leu Val Ala Ile Asp Glu Leu Met Gln Ser Leu Asn His
                485                 490                 495

Asn Gly Glu Thr Leu Arg Gln Lys Pro Pro Val Gly Glu Ala Asp Pro
            500                 505                 510

Tyr Arg Val Lys Met Lys Leu Cys Ile Leu Leu His Ala Phe Ser Thr
        515                 520                 525

Arg Val Val Thr Ile Asn Arg Val Met Gly Tyr Leu Ser Ser Ala Lys
    530                 535                 540

Val Asp Lys Arg Val Glu Ser Lys Tyr Gly Pro Pro Cys Pro Pro Cys
545                 550                 555                 560

Pro Ala Pro Glu Phe Leu Gly Gly Pro Ser Val Phe Leu Phe Pro Pro
                565                 570                 575

Lys Pro Lys Asp Thr Leu Met Asn Ser Arg Thr Pro Glu Val Thr Cys
            580                 585                 590

Val Val Val Asp Val Ser Gln Glu Asp Pro Glu Val Gln Phe Asn Trp
        595                 600                 605

Tyr Val Asp Gly Val Glu Val His Asn Ala Lys Thr Lys Pro Arg Glu
    610                 615                 620

Glu Gln Phe Asn Ser Thr Tyr Arg Val Val Ser Val Leu Thr Val Leu
625                 630                 635                 640

His Gln Asp Trp Leu Asn Gly Lys Glu Tyr Lys Cys Lys Val Ser Asn
                645                 650                 655

Lys Gly Leu Pro Ser Ser Ile Glu Lys Thr Ile Ser Lys Ala Lys Gly
            660                 665                 670

Gln Pro Arg Glu Pro Gln Val Tyr Thr Leu Pro Pro Ser Pro Glu Glu
        675                 680                 685

Met Thr Lys Asn Gln Val Ser Leu Ser Cys Ala Val Lys Gly Phe Tyr
    690                 695                 700

Pro Ser Asp Ile Ala Val Glu Trp Glu Ser Asn Gly Gln Pro Glu Asn

705                 710                 715                 720

Asn Tyr Lys Thr Thr Pro Pro Val Leu Asp Ser Asp Gly Ser Phe Phe
                725                 730                 735

Leu Val Ser Arg Leu Thr Val Asp Lys Ser Arg Trp Gln Glu Gly Asn
            740                 745                 750

Val Phe Ser Cys Ser Val Met His Glu Ala Leu His Asn Gln Tyr Thr
        755                 760                 765

Gln Lys Ser Leu Ser Leu Ser Leu Gly Lys
    770                 775

<210>  16
<211>  775
<212>  PRT
<213>  Homo sapiens

<400>  16

Met Cys His Gln Gln Leu Val Ile Ser Trp Phe Ser Leu Val Phe Leu
1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu
        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp
            100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg

145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser Gly Gly Gly Gly Ser Gly Gly Gly
                325                 330                 335

Gly Ser Gly Gly Gly Gly Ser Arg Asn Leu Pro Val Ala Thr Pro Asp
            340                 345                 350

Pro Gly Met Phe Pro Cys Leu His His Ser Gln Asn Leu Leu Arg Ala
        355                 360                 365

Val Ser Asn Met Leu Gln Lys Ala Arg Gln Thr Leu Glu Phe Tyr Pro
    370                 375                 380

Cys Thr Ser Glu Glu Ile Asp His Glu Asp Ile Thr Lys Asp Lys Thr
385                 390                 395                 400

Ser Thr Val Glu Ala Cys Leu Pro Leu Glu Leu Thr Lys Asn Glu Ser
                405                 410                 415

Cys Leu Asn Ser Arg Glu Thr Ser Phe Ile Thr Asn Gly Ser Cys Leu
            420                 425                 430

Ala Ser Arg Lys Thr Ser Phe Met Met Ala Leu Cys Leu Ser Ser Ile
        435                 440                 445

Tyr Glu Asp Leu Lys Met Tyr Gln Val Glu Phe Lys Thr Met Asn Ala
    450                 455                 460

Lys Leu Leu Met Asp Pro Lys Arg Gln Ile Phe Leu Asp Gln Asn Met
465                 470                 475                 480

Leu Ala Val Ile Asp Glu Leu Met Gln Ala Leu Asn Phe Asn Ser Glu
                485                 490                 495

Thr Val Pro Gln Lys Ser Ser Leu Glu Glu Pro Asp Phe Tyr Lys Thr
            500                 505                 510

Lys Ile Lys Leu Cys Ile Leu Leu His Ala Phe Arg Ile Arg Ala Val
        515                 520                 525

Thr Ile Asp Arg Val Met Ser Tyr Leu Asn Ala Ser Lys Val Asp Lys
    530                 535                 540

Arg Val Glu Ser Lys Tyr Gly Pro Pro Cys Pro Pro Cys Pro Ala Pro
545                 550                 555                 560

Glu Phe Leu Gly Gly Pro Ser Val Phe Leu Phe Pro Pro Lys Pro Lys
                565                 570                 575

Asp Thr Leu Met Asn Ser Arg Thr Pro Glu Val Thr Cys Val Val Val
            580                 585                 590

Asp Val Ser Gln Glu Asp Pro Glu Val Gln Phe Asn Trp Tyr Val Asp
        595                 600                 605

Gly Val Glu Val His Asn Ala Lys Thr Lys Pro Arg Glu Glu Gln Phe
    610                 615                 620

Asn Ser Thr Tyr Arg Val Val Ser Val Leu Thr Val Leu His Gln Asp
625                 630                 635                 640

Trp Leu Asn Gly Lys Glu Tyr Lys Cys Lys Val Ser Asn Lys Gly Leu
                645                 650                 655

Pro Ser Ser Ile Glu Lys Thr Ile Ser Lys Ala Lys Gly Gln Pro Arg
            660                 665                 670

Glu Pro Gln Val Tyr Thr Leu Pro Pro Ser Pro Glu Glu Met Thr Lys
        675                 680                 685

Asn Gln Val Ser Leu Ser Cys Ala Val Lys Gly Phe Tyr Pro Ser Asp
    690                 695                 700

Ile Ala Val Glu Trp Glu Ser Asn Gly Gln Pro Glu Asn Asn Tyr Lys
705                 710                 715                 720

Thr Thr Pro Pro Val Leu Asp Ser Asp Gly Ser Phe Phe Leu Val Ser
                725                 730                 735

Arg Leu Thr Val Asp Lys Ser Arg Trp Gln Glu Gly Asn Val Phe Ser
            740                 745                 750

Cys Ser Val Met His Glu Ala Leu His Asn Gln Tyr Thr Gln Lys Ser
        755                 760                 765

Leu Ser Leu Ser Leu Gly Lys
    770                 775

<210> 17
<211> 781
<212> PRT
<213> Mus musculus

<400> 17

Met Cys Pro Gln Lys Leu Thr Ile Ser Trp Phe Ala Ile Val Leu Leu
1               5                   10                  15

Val Ser Pro Leu Met Ala Met Trp Glu Leu Glu Lys Asp Val Tyr Val
            20                  25                  30

Val Glu Val Asp Trp Thr Pro Asp Ala Pro Gly Glu Thr Val Asn Leu
        35                  40                  45

Thr Cys Asp Thr Pro Glu Glu Asp Asp Ile Thr Trp Thr Ser Asp Gln
    50                  55                  60

Arg His Gly Val Ile Gly Ser Gly Lys Thr Leu Thr Ile Thr Val Lys
65                  70                  75                  80

Glu Phe Leu Asp Ala Gly Gln Tyr Thr Cys His Lys Gly Gly Glu Thr
                85                  90                  95

Leu Ser His Ser His Leu Leu Leu His Lys Lys Glu Asn Gly Ile Trp
        100                 105                 110

Ser Thr Glu Ile Leu Lys Asn Phe Lys Asn Lys Thr Phe Leu Lys Cys
    115                 120                 125

Glu Ala Pro Asn Tyr Ser Gly Arg Phe Thr Cys Ser Trp Leu Val Gln
    130                 135                 140

Arg Asn Met Asp Leu Lys Phe Asn Ile Lys Ser Ser Ser Ser Ser Pro
145                 150                 155                 160

Asp Ser Arg Ala Val Thr Cys Gly Met Ala Ser Leu Ser Ala Glu Lys
                165                 170                 175

Val Thr Leu Asp Gln Arg Asp Tyr Glu Lys Tyr Ser Val Ser Cys Gln
            180                 185                 190

Glu Asp Val Thr Cys Pro Thr Ala Glu Glu Thr Leu Pro Ile Glu Leu
        195                 200                 205

Ala Leu Glu Ala Arg Gln Gln Asn Lys Tyr Glu Asn Tyr Ser Thr Ser
    210                 215                 220

Phe Phe Ile Arg Asp Ile Ile Lys Pro Asp Pro Pro Lys Asn Leu Gln
225                 230                 235                 240

Met Lys Pro Leu Lys Asn Ser Gln Val Glu Val Ser Trp Glu Tyr Pro
                245                 250                 255

Asp Ser Trp Ser Thr Pro His Ser Tyr Phe Ser Leu Lys Phe Phe Val
            260                 265                 270

Arg Ile Gln Arg Lys Lys Glu Lys Met Lys Glu Thr Glu Glu Gly Cys
        275                 280                 285

Asn Gln Lys Gly Ala Phe Leu Val Glu Lys Thr Ser Thr Glu Val Gln
    290                 295                 300

Cys Lys Gly Gly Asn Val Cys Val Gln Ala Gln Asp Arg Tyr Tyr Asn
305                 310                 315                 320

Ser Ser Cys Ser Lys Trp Ala Cys Val Pro Cys Arg Val Arg Ser Gly
                325                 330                 335

Gly Gly Gly Ser Gly Gly Gly Gly Ser Gly Gly Gly Gly Ser Arg Val
            340                 345                 350

Ile Pro Val Ser Gly Pro Ala Arg Cys Leu Ser Gln Ser Arg Asn Leu
        355                 360                 365

Leu Lys Thr Thr Asp Asp Met Val Lys Thr Ala Arg Glu Lys Leu Lys
    370                 375                 380

His Tyr Ser Cys Thr Ala Glu Asp Ile Asp His Glu Asp Ile Thr Arg
385                 390                 395                 400

Asp Gln Thr Ser Thr Leu Lys Thr Cys Leu Pro Leu Glu Leu His Lys
                405                 410                 415

Asn Glu Ser Cys Leu Ala Thr Arg Glu Thr Ser Ser Thr Thr Arg Gly
            420                 425                 430

Ser Cys Leu Pro Pro Gln Lys Thr Ser Leu Met Met Thr Leu Cys Leu
        435                 440                 445

Gly Ser Ile Tyr Glu Asp Leu Lys Met Tyr Gln Thr Glu Phe Gln Ala
    450                 455                 460

Ile Asn Ala Ala Leu Gln Asn His Asn His Gln Gln Ile Ile Leu Asp
465                 470                 475                 480

Lys Gly Met Leu Val Ala Ile Asp Glu Leu Met Gln Ser Leu Asn His
                485                 490                 495

Asn Gly Glu Thr Leu Arg Gln Lys Pro Pro Val Gly Glu Ala Asp Pro
            500                 505                 510

Tyr Arg Val Lys Met Lys Leu Cys Ile Leu Leu His Ala Phe Ser Thr
        515                 520                 525

Arg Val Val Thr Ile Asn Arg Val Met Gly Tyr Leu Ser Ser Ala Lys
    530                 535                 540

Val Asp Lys Lys Val Glu Pro Lys Ser Cys Asp Lys Thr His Thr Cys
545                 550                 555                 560

Pro Pro Cys Pro Ala Pro Glu Leu Leu Gly Gly Pro Ser Val Phe Leu
                565                 570                 575

Phe Pro Pro Lys Pro Lys Asp Thr Leu Met Asn Ser Arg Thr Pro Glu
            580                 585                 590

Val Thr Cys Val Val Val Asp Val Ser His Glu Asp Pro Glu Val Lys

595                 600                 605

Phe Asn Trp Tyr Val Asp Gly Val Glu Val His Asn Ala Lys Thr Lys
    610                 615                 620

Pro Arg Glu Glu Gln Tyr Ala Ser Thr Tyr Arg Val Val Ser Val Leu
625                 630                 635                 640

Thr Val Leu His Gln Asp Trp Leu Asn Gly Lys Glu Tyr Lys Cys Lys
                645                 650                 655

Val Ser Asn Lys Ala Leu Pro Ala Pro Ile Glu Lys Thr Ile Ser Lys
            660                 665                 670

Ala Lys Gly Gln Pro Arg Glu Pro Gln Val Tyr Thr Leu Pro Pro Ser
        675                 680                 685

Arg Glu Glu Met Thr Lys Asn Gln Val Ser Leu Ser Cys Ala Val Lys
    690                 695                 700

Gly Phe Tyr Pro Ser Asp Ile Ala Val Glu Trp Glu Ser Asn Gly Gln
705                 710                 715                 720

Pro Glu Asn Asn Tyr Lys Thr Thr Pro Pro Val Leu Asp Ser Asp Gly
                725                 730                 735

Ser Phe Phe Leu Val Ser Lys Leu Thr Val Asp Lys Ser Arg Trp Gln
            740                 745                 750

Gln Gly Asn Val Phe Ser Cys Ser Val Met His Glu Ala Leu His Asn
        755                 760                 765

Gln Tyr Thr Gln Lys Ser Leu Ser Leu Ser Pro Gly Lys
    770                 775                 780

<210>  18
<211>  778
<212>  PRT
<213>  Homo sapiens

<400>  18

Met Cys His Gln Gln Leu Val Ile Ser Trp Phe Ser Leu Val Phe Leu
1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu

        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp
            100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg
145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser Gly Gly Gly Gly Ser Gly Gly Gly
                325                 330                 335

Gly Ser Gly Gly Gly Gly Ser Arg Asn Leu Pro Val Ala Thr Pro Asp
            340                 345                 350

Pro Gly Met Phe Pro Cys Leu His His Ser Gln Asn Leu Leu Arg Ala
        355                 360                 365

Val Ser Asn Met Leu Gln Lys Ala Arg Gln Thr Leu Glu Phe Tyr Pro
    370                 375                 380

Cys Thr Ser Glu Glu Ile Asp His Glu Asp Ile Thr Lys Asp Lys Thr
385                 390                 395                 400

Ser Thr Val Glu Ala Cys Leu Pro Leu Glu Leu Thr Lys Asn Glu Ser
                405                 410                 415

Cys Leu Asn Ser Arg Glu Thr Ser Phe Ile Thr Asn Gly Ser Cys Leu
            420                 425                 430

Ala Ser Arg Lys Thr Ser Phe Met Met Ala Leu Cys Leu Ser Ser Ile
        435                 440                 445

Tyr Glu Asp Leu Lys Met Tyr Gln Val Glu Phe Lys Thr Met Asn Ala
    450                 455                 460

Lys Leu Leu Met Asp Pro Lys Arg Gln Ile Phe Leu Asp Gln Asn Met
465                 470                 475                 480

Leu Ala Val Ile Asp Glu Leu Met Gln Ala Leu Asn Phe Asn Ser Glu
                485                 490                 495

Thr Val Pro Gln Lys Ser Ser Leu Glu Glu Pro Asp Phe Tyr Lys Thr
            500                 505                 510

Lys Ile Lys Leu Cys Ile Leu Leu His Ala Phe Arg Ile Arg Ala Val
        515                 520                 525

Thr Ile Asp Arg Val Met Ser Tyr Leu Asn Ala Ser Lys Val Asp Lys
    530                 535                 540

Lys Val Glu Pro Lys Ser Cys Asp Lys Thr His Thr Cys Pro Pro Cys
545                 550                 555                 560

Pro Ala Pro Glu Leu Leu Gly Gly Pro Ser Val Phe Leu Phe Pro Pro
                565                 570                 575

Lys Pro Lys Asp Thr Leu Met Asn Ser Arg Thr Pro Glu Val Thr Cys
            580                 585                 590

Val Val Val Asp Val Ser His Glu Asp Pro Glu Val Lys Phe Asn Trp
        595                 600                 605

Tyr Val Asp Gly Val Glu Val His Asn Ala Lys Thr Lys Pro Arg Glu
    610                 615                 620

Glu Gln Tyr Ala Ser Thr Tyr Arg Val Val Ser Val Leu Thr Val Leu
625                 630                 635                 640

His Gln Asp Trp Leu Asn Gly Lys Glu Tyr Lys Cys Lys Val Ser Asn
                645                 650                 655

Lys Ala Leu Pro Ala Pro Ile Glu Lys Thr Ile Ser Lys Ala Lys Gly
            660                 665                 670

Gln Pro Arg Glu Pro Gln Val Tyr Thr Leu Pro Pro Ser Arg Glu Glu
        675                 680                 685

Met Thr Lys Asn Gln Val Ser Leu Ser Cys Ala Val Lys Gly Phe Tyr
    690                 695                 700

Pro Ser Asp Ile Ala Val Glu Trp Glu Ser Asn Gly Gln Pro Glu Asn
705                 710                 715                 720

Asn Tyr Lys Thr Thr Pro Pro Val Leu Asp Ser Asp Gly Ser Phe Phe
                725                 730                 735

Leu Val Ser Lys Leu Thr Val Asp Lys Ser Arg Trp Gln Gln Gly Asn
            740                 745                 750

Val Phe Ser Cys Ser Val Met His Glu Ala Leu His Asn Gln Tyr Thr
        755                 760                 765

Gln Lys Ser Leu Ser Leu Ser Pro Gly Lys
    770                 775

<210>  19
<211>  873

<212>  PRT
<213>  Mus musculus

<400>  19

Met Cys Pro Gln Lys Leu Thr Ile Ser Trp Phe Ala Ile Val Leu Leu
1               5                   10                  15

Val Ser Pro Leu Met Ala Met Trp Glu Leu Glu Lys Asp Val Tyr Val
            20                  25                  30

Val Glu Val Asp Trp Thr Pro Asp Ala Pro Gly Glu Thr Val Asn Leu
        35                  40                  45

Thr Cys Asp Thr Pro Glu Glu Asp Asp Ile Thr Trp Thr Ser Asp Gln
    50                  55                  60

Arg His Gly Val Ile Gly Ser Gly Lys Thr Leu Thr Ile Thr Val Lys
65                  70                  75                  80

Glu Phe Leu Asp Ala Gly Gln Tyr Thr Cys His Lys Gly Gly Glu Thr
                85                  90                  95

Leu Ser His Ser His Leu Leu Leu His Lys Lys Glu Asn Gly Ile Trp
            100                 105                 110

Ser Thr Glu Ile Leu Lys Asn Phe Lys Asn Lys Thr Phe Leu Lys Cys
        115                 120                 125

Glu Ala Pro Asn Tyr Ser Gly Arg Phe Thr Cys Ser Trp Leu Val Gln
    130                 135                 140

Arg Asn Met Asp Leu Lys Phe Asn Ile Lys Ser Ser Ser Ser Ser Pro
145                 150                 155                 160

Asp Ser Arg Ala Val Thr Cys Gly Met Ala Ser Leu Ser Ala Glu Lys
                165                 170                 175

Val Thr Leu Asp Gln Arg Asp Tyr Glu Lys Tyr Ser Val Ser Cys Gln
            180                 185                 190

Glu Asp Val Thr Cys Pro Thr Ala Glu Glu Thr Leu Pro Ile Glu Leu
        195                 200                 205

Ala Leu Glu Ala Arg Gln Gln Asn Lys Tyr Glu Asn Tyr Ser Thr Ser
    210                 215                 220

Phe Phe Ile Arg Asp Ile Ile Lys Pro Asp Pro Pro Lys Asn Leu Gln
225                 230                 235                 240

Met Lys Pro Leu Lys Asn Ser Gln Val Glu Val Ser Trp Glu Tyr Pro
                245                 250                 255

Asp Ser Trp Ser Thr Pro His Ser Tyr Phe Ser Leu Lys Phe Phe Val
            260                 265                 270

Arg Ile Gln Arg Lys Lys Glu Lys Met Lys Glu Thr Glu Glu Gly Cys
        275                 280                 285

Asn Gln Lys Gly Ala Phe Leu Val Glu Lys Thr Ser Thr Glu Val Gln
    290                 295                 300

Cys Lys Gly Gly Asn Val Cys Val Gln Ala Gln Asp Arg Tyr Tyr Asn
305                 310                 315                 320

Ser Ser Cys Ser Lys Trp Ala Cys Val Pro Cys Arg Val Arg Ser Gly
                325                 330                 335

Gly Gly Gly Ser Gly Gly Gly Gly Ser Gly Gly Gly Gly Ser Arg Val
            340                 345                 350

Ile Pro Val Ser Gly Pro Ala Arg Cys Leu Ser Gln Ser Arg Asn Leu
        355                 360                 365

Leu Lys Thr Thr Asp Asp Met Val Lys Thr Ala Arg Glu Lys Leu Lys
    370                 375                 380

His Tyr Ser Cys Thr Ala Glu Asp Ile Asp His Glu Asp Ile Thr Arg
385                 390                 395                 400

Asp Gln Thr Ser Thr Leu Lys Thr Cys Leu Pro Leu Glu Leu His Lys
                405                 410                 415

Asn Glu Ser Cys Leu Ala Thr Arg Glu Thr Ser Ser Thr Thr Arg Gly
            420                 425                 430

Ser Cys Leu Pro Pro Gln Lys Thr Ser Leu Met Met Thr Leu Cys Leu
        435                 440                 445

Gly Ser Ile Tyr Glu Asp Leu Lys Met Tyr Gln Thr Glu Phe Gln Ala
    450                 455                 460

Ile Asn Ala Ala Leu Gln Asn His Asn His Gln Gln Ile Ile Leu Asp
465                 470                 475                 480

Lys Gly Met Leu Val Ala Ile Asp Glu Leu Met Gln Ser Leu Asn His

485                 490                 495

Asn Gly Glu Thr Leu Arg Gln Lys Pro Pro Val Gly Glu Ala Asp Pro
            500                 505                 510

Tyr Arg Val Lys Met Lys Leu Cys Ile Leu Leu His Ala Phe Ser Thr
        515                 520                 525

Arg Val Val Thr Ile Asn Arg Val Met Gly Tyr Leu Ser Ser Ala Ala
    530                 535                 540

Ser Thr Lys Gly Pro Ser Val Phe Pro Leu Ala Pro Ser Ser Lys Ser
545                 550                 555                 560

Thr Ser Gly Gly Thr Ala Ala Leu Gly Cys Leu Val Lys Asp Tyr Phe
                565                 570                 575

Pro Glu Pro Val Thr Val Ser Trp Asn Ser Gly Ala Leu Thr Ser Gly
            580                 585                 590

Val His Thr Phe Pro Ala Val Leu Gln Ser Ser Gly Leu Tyr Ser Leu
        595                 600                 605

Ser Ser Val Val Thr Val Pro Ser Ser Ser Leu Gly Thr Gln Thr Tyr
    610                 615                 620

Ile Cys Asn Val Asn His Lys Pro Ser Asn Thr Lys Val Asp Lys Lys
625                 630                 635                 640

Val Glu Pro Lys Ser Cys Asp Lys Thr His Thr Cys Pro Pro Cys Pro
                645                 650                 655

Ala Pro Glu Leu Leu Gly Gly Pro Ser Val Phe Leu Phe Pro Pro Lys
            660                 665                 670

Pro Lys Asp Thr Leu Met Asn Ser Arg Thr Pro Glu Val Thr Cys Val
        675                 680                 685

Val Val Asp Val Ser His Glu Asp Pro Glu Val Lys Phe Asn Trp Tyr
    690                 695                 700

Val Asp Gly Val Glu Val His Asn Ala Lys Thr Lys Pro Arg Glu Glu
705                 710                 715                 720

Gln Tyr Ala Ser Thr Tyr Arg Val Val Ser Val Leu Thr Val Leu His
                725                 730                 735

Gln Asp Trp Leu Asn Gly Lys Glu Tyr Lys Cys Lys Val Ser Asn Lys
            740                 745                 750

Ala Leu Pro Ala Pro Ile Glu Lys Thr Ile Ser Lys Ala Lys Gly Gln
        755                 760                 765

Pro Arg Glu Pro Gln Val Tyr Thr Leu Pro Pro Ser Arg Glu Glu Met
    770                 775                 780

Thr Lys Asn Gln Val Ser Leu Ser Cys Ala Val Lys Gly Phe Tyr Pro
785                 790                 795                 800

Ser Asp Ile Ala Val Glu Trp Glu Ser Asn Gly Gln Pro Glu Asn Asn
                805                 810                 815

Tyr Lys Thr Thr Pro Pro Val Leu Asp Ser Asp Gly Ser Phe Phe Leu
            820                 825                 830

Val Ser Lys Leu Thr Val Asp Lys Ser Arg Trp Gln Gln Gly Asn Val
        835                 840                 845

Phe Ser Cys Ser Val Met His Glu Ala Leu His Asn Gln Tyr Thr Gln
    850                 855                 860

Lys Ser Leu Ser Leu Ser Pro Gly Lys
865                 870

<210>  20
<211>  870
<212>  PRT
<213>  Homo sapiens

<400>  20

Met Cys His Gln Gln Leu Val Ile Ser Trp Phe Ser Leu Val Phe Leu
1               5                   10                  15

Ala Ser Pro Leu Val Ala Ile Trp Glu Leu Lys Lys Asp Val Tyr Val
            20                  25                  30

Val Glu Leu Asp Trp Tyr Pro Asp Ala Pro Gly Glu Met Val Val Leu
        35                  40                  45

Thr Cys Asp Thr Pro Glu Glu Asp Gly Ile Thr Trp Thr Leu Asp Gln
    50                  55                  60

Ser Ser Glu Val Leu Gly Ser Gly Lys Thr Leu Thr Ile Gln Val Lys
65                  70                  75                  80

Glu Phe Gly Asp Ala Gly Gln Tyr Thr Cys His Lys Gly Gly Glu Val
                85                  90                  95

Leu Ser His Ser Leu Leu Leu Leu His Lys Lys Glu Asp Gly Ile Trp
            100                 105                 110

Ser Thr Asp Ile Leu Lys Asp Gln Lys Glu Pro Lys Asn Lys Thr Phe
        115                 120                 125

Leu Arg Cys Glu Ala Lys Asn Tyr Ser Gly Arg Phe Thr Cys Trp Trp
    130                 135                 140

Leu Thr Thr Ile Ser Thr Asp Leu Thr Phe Ser Val Lys Ser Ser Arg
145                 150                 155                 160

Gly Ser Ser Asp Pro Gln Gly Val Thr Cys Gly Ala Ala Thr Leu Ser
                165                 170                 175

Ala Glu Arg Val Arg Gly Asp Asn Lys Glu Tyr Glu Tyr Ser Val Glu
            180                 185                 190

Cys Gln Glu Asp Ser Ala Cys Pro Ala Ala Glu Glu Ser Leu Pro Ile
        195                 200                 205

Glu Val Met Val Asp Ala Val His Lys Leu Lys Tyr Glu Asn Tyr Thr
    210                 215                 220

Ser Ser Phe Phe Ile Arg Asp Ile Ile Lys Pro Asp Pro Pro Lys Asn
225                 230                 235                 240

Leu Gln Leu Lys Pro Leu Lys Asn Ser Arg Gln Val Glu Val Ser Trp
                245                 250                 255

Glu Tyr Pro Asp Thr Trp Ser Thr Pro His Ser Tyr Phe Ser Leu Thr
            260                 265                 270

Phe Cys Val Gln Val Gln Gly Lys Ser Lys Arg Glu Lys Lys Asp Arg
        275                 280                 285

Val Phe Thr Asp Lys Thr Ser Ala Thr Val Ile Cys Arg Lys Asn Ala
    290                 295                 300

Ser Ile Ser Val Arg Ala Gln Asp Arg Tyr Tyr Ser Ser Ser Trp Ser
305                 310                 315                 320

Glu Trp Ala Ser Val Pro Cys Ser Gly Gly Gly Gly Ser Gly Gly Gly
                325                 330                 335

Gly Ser Gly Gly Gly Gly Ser Arg Asn Leu Pro Val Ala Thr Pro Asp
        340                 345                 350

Pro Gly Met Phe Pro Cys Leu His His Ser Gln Asn Leu Leu Arg Ala
    355                 360                 365

Val Ser Asn Met Leu Gln Lys Ala Arg Gln Thr Leu Glu Phe Tyr Pro
    370                 375                 380

Cys Thr Ser Glu Glu Ile Asp His Glu Asp Ile Thr Lys Asp Lys Thr
385                 390                 395                 400

Ser Thr Val Glu Ala Cys Leu Pro Leu Glu Leu Thr Lys Asn Glu Ser
                405                 410                 415

Cys Leu Asn Ser Arg Glu Thr Ser Phe Ile Thr Asn Gly Ser Cys Leu
            420                 425                 430

Ala Ser Arg Lys Thr Ser Phe Met Met Ala Leu Cys Leu Ser Ser Ile
        435                 440                 445

Tyr Glu Asp Leu Lys Met Tyr Gln Val Glu Phe Lys Thr Met Asn Ala
    450                 455                 460

Lys Leu Leu Met Asp Pro Lys Arg Gln Ile Phe Leu Asp Gln Asn Met
465                 470                 475                 480

Leu Ala Val Ile Asp Glu Leu Met Gln Ala Leu Asn Phe Asn Ser Glu
                485                 490                 495

Thr Val Pro Gln Lys Ser Ser Leu Glu Glu Pro Asp Phe Tyr Lys Thr
            500                 505                 510

Lys Ile Lys Leu Cys Ile Leu Leu His Ala Phe Arg Ile Arg Ala Val
        515                 520                 525

Thr Ile Asp Arg Val Met Ser Tyr Leu Asn Ala Ser Ala Ser Thr Lys
    530                 535                 540

Gly Pro Ser Val Phe Pro Leu Ala Pro Ser Ser Lys Ser Thr Ser Gly
545                 550                 555                 560

Gly Thr Ala Ala Leu Gly Cys Leu Val Lys Asp Tyr Phe Pro Glu Pro
                565                 570                 575

Val Thr Val Ser Trp Asn Ser Gly Ala Leu Thr Ser Gly Val His Thr
            580                 585                 590

Phe Pro Ala Val Leu Gln Ser Ser Gly Leu Tyr Ser Leu Ser Ser Val
        595                 600                 605

Val Thr Val Pro Ser Ser Ser Leu Gly Thr Gln Thr Tyr Ile Cys Asn
    610                 615                 620

Val Asn His Lys Pro Ser Asn Thr Lys Val Asp Lys Lys Val Glu Pro
625                 630                 635                 640

Lys Ser Cys Asp Lys Thr His Thr Cys Pro Pro Cys Pro Ala Pro Glu
                645                 650                 655

Leu Leu Gly Gly Pro Ser Val Phe Leu Phe Pro Pro Lys Pro Lys Asp
            660                 665                 670

Thr Leu Met Asn Ser Arg Thr Pro Glu Val Thr Cys Val Val Val Asp
        675                 680                 685

Val Ser His Glu Asp Pro Glu Val Lys Phe Asn Trp Tyr Val Asp Gly
    690                 695                 700

Val Glu Val His Asn Ala Lys Thr Lys Pro Arg Glu Glu Gln Tyr Ala
705                 710                 715                 720

Ser Thr Tyr Arg Val Val Ser Val Leu Thr Val Leu His Gln Asp Trp
                725                 730                 735

Leu Asn Gly Lys Glu Tyr Lys Cys Lys Val Ser Asn Lys Ala Leu Pro
            740                 745                 750

Ala Pro Ile Glu Lys Thr Ile Ser Lys Ala Lys Gly Gln Pro Arg Glu
        755                 760                 765

Pro Gln Val Tyr Thr Leu Pro Pro Ser Arg Glu Glu Met Thr Lys Asn
    770                 775                 780

Gln Val Ser Leu Ser Cys Ala Val Lys Gly Phe Tyr Pro Ser Asp Ile
785                 790                 795                 800

Ala Val Glu Trp Glu Ser Asn Gly Gln Pro Glu Asn Asn Tyr Lys Thr
                805                 810                 815

Thr Pro Pro Val Leu Asp Ser Asp Gly Ser Phe Phe Leu Val Ser Lys
            820                 825                 830

Leu Thr Val Asp Lys Ser Arg Trp Gln Gln Gly Asn Val Phe Ser Cys

835                 840                 845

Ser Val Met His Glu Ala Leu His Asn Gln Tyr Thr Gln Lys Ser Leu
    850                 855                 860

Ser Leu Ser Pro Gly Lys
865                 870

<210>  21
<211>  596
<212>  PRT
<213>  Artificial Sequence

<220>
<223>  fusion protein

<400>  21

Met Lys Tyr Leu Leu Pro Thr Ala Ala Ala Gly Leu Leu Leu Leu Ala
1               5                   10                  15

Ala Gln Pro Ala Met Ala Asp Asp Ile Gln Met Thr Gln Ser Pro Ser
            20                  25                  30

Ser Leu Ser Ala Ser Val Gly Asp Arg Val Thr Ile Thr Cys Arg Ala
        35                  40                  45

Ser Gln Asp Val Ser Thr Ala Val Ala Trp Tyr Gln Gln Lys Pro Gly
    50                  55                  60

Lys Ala Pro Lys Leu Leu Ile Tyr Ser Ala Ser Phe Leu Tyr Ser Gly
65                  70                  75                  80

Val Pro Ser Arg Phe Ser Gly Ser Gly Ser Gly Thr Asp Phe Thr Leu
                85                  90                  95

Thr Ile Ser Ser Leu Gln Pro Glu Asp Phe Ala Thr Tyr Tyr Cys Gln
            100                 105                 110

Gln Tyr Leu Tyr His Pro Ala Thr Phe Gly Gln Gly Thr Lys Val Glu
        115                 120                 125

Ile Lys Arg Gly Gly Gly Gly Ser Gly Gly Gly Gly Ser Gly Gly Gly
    130                 135                 140

Gly Ser Gly Gly Gly Gly Ser Glu Val Gln Leu Val Glu Ser Gly Gly
145                 150                 155                 160

Gly Leu Val Gln Pro Gly Gly Ser Leu Arg Leu Ser Cys Ala Ala Ser
                165                 170                 175

Gly Phe Thr Phe Ser Asp Ser Trp Ile His Trp Val Arg Gln Ala Pro
            180                 185                 190

Gly Lys Gly Leu Glu Trp Val Ala Trp Ile Ser Pro Tyr Gly Gly Ser
        195                 200                 205

Thr Tyr Tyr Ala Asp Ser Val Lys Gly Arg Phe Thr Ile Ser Ala Asp
    210                 215                 220

Thr Ser Lys Asn Thr Ala Tyr Leu Gln Met Asn Ser Leu Arg Ala Glu
225                 230                 235                 240

Asp Thr Ala Val Tyr Tyr Cys Ala Arg Arg His Trp Pro Gly Gly Phe
                245                 250                 255

Asp Tyr Trp Gly Gln Gly Thr Leu Val Thr Val Ser Ala Ala Ser Thr
            260                 265                 270

Lys Gly Pro Ser Val Phe Pro Leu Ala Pro Cys Ser Arg Ser Thr Ser
        275                 280                 285

Glu Ser Thr Ala Ala Leu Gly Cys Leu Val Lys Asp Tyr Phe Pro Glu
    290                 295                 300

Pro Val Thr Val Ser Trp Asn Ser Gly Ala Leu Thr Ser Gly Val His
305                 310                 315                 320

Thr Phe Pro Ala Val Leu Gln Ser Ser Gly Leu Tyr Ser Leu Ser Ser
                325                 330                 335

Val Val Thr Val Pro Ser Ser Ser Leu Gly Thr Lys Thr Tyr Thr Cys
            340                 345                 350

Asn Val Asp His Lys Pro Ser Asn Thr Lys Val Asp Lys Arg Val Glu
        355                 360                 365

Ser Lys Tyr Gly Pro Pro Cys Pro Pro Cys Pro Ala Pro Glu Phe Leu
    370                 375                 380

Gly Gly Pro Ser Val Phe Leu Phe Pro Pro Lys Pro Lys Asp Thr Leu
385                 390                 395                 400

Met Asn Ser Arg Thr Pro Glu Val Thr Cys Val Val Val Asp Val Ser
                405                 410                 415

Gln Glu Asp Pro Glu Val Gln Phe Asn Trp Tyr Val Asp Gly Val Glu

420                 425                 430

Val His Asn Ala Lys Thr Lys Pro Arg Glu Glu Gln Phe Asn Ser Thr
        435                 440                 445

Tyr Arg Val Val Ser Val Leu Thr Val Leu His Gln Asp Trp Leu Asn
    450                 455                 460

Gly Lys Glu Tyr Lys Cys Lys Val Ser Asn Lys Gly Leu Pro Ser Ser
465                 470                 475                 480

Ile Glu Lys Thr Ile Ser Lys Ala Lys Gly Gln Pro Arg Glu Pro Gln
                485                 490                 495

Val Tyr Thr Leu Pro Pro Ser Pro Glu Glu Met Thr Lys Asn Gln Val
            500                 505                 510

Ser Leu Trp Cys Leu Val Lys Gly Phe Tyr Pro Ser Asp Ile Ala Val
        515                 520                 525

Glu Trp Glu Ser Asn Gly Gln Pro Glu Asn Asn Tyr Lys Thr Thr Pro
    530                 535                 540

Pro Val Leu Asp Ser Asp Gly Ser Phe Phe Leu Tyr Ser Arg Leu Thr
545                 550                 555                 560

Val Asp Lys Ser Arg Trp Gln Glu Gly Asn Val Phe Ser Cys Ser Val
                565                 570                 575

Met His Glu Ala Leu His Asn Gln Tyr Thr Gln Lys Ser Leu Ser Leu
            580                 585                 590

Ser Leu Gly Lys
        595

<210>  22
<211>  598
<212>  PRT
<213>  Artificial Sequence

<220>
<223>  fusion protein

<400>  22

Met Lys Tyr Leu Leu Pro Thr Ala Ala Ala Gly Leu Leu Leu Leu Ala
1               5                   10                  15

Ala Gln Pro Ala Met Ala Asp Ile Gln Met Thr Gln Ser Pro Ser Ser
            20                  25                  30

Leu Ser Ala Ser Val Gly Asp Arg Val Thr Ile Thr Cys Arg Ala Ser
        35                  40                  45

Gln Asp Val Ser Thr Ala Val Ala Trp Tyr Gln Gln Lys Pro Gly Lys
    50                  55                  60

Ala Pro Lys Leu Leu Ile Tyr Ser Ala Ser Phe Leu Tyr Ser Gly Val
65                  70                  75                  80

Pro Ser Arg Phe Ser Gly Ser Gly Ser Gly Thr Asp Phe Thr Leu Thr
                85                  90                  95

Ile Ser Ser Leu Gln Pro Glu Asp Phe Ala Thr Tyr Tyr Cys Gln Gln
            100                 105                 110

Tyr Leu Tyr His Pro Ala Thr Phe Gly Gln Gly Thr Lys Val Glu Ile
        115                 120                 125

Lys Arg Gly Gly Gly Gly Ser Gly Gly Gly Gly Ser Gly Gly Gly Gly
    130                 135                 140

Ser Gly Gly Gly Gly Ser Glu Val Gln Leu Val Glu Ser Gly Gly Gly
145                 150                 155                 160

Leu Val Gln Pro Gly Gly Ser Leu Arg Leu Ser Cys Ala Ala Ser Gly
                165                 170                 175

Phe Thr Phe Ser Asp Ser Trp Ile His Trp Val Arg Gln Ala Pro Gly
            180                 185                 190

Lys Gly Leu Glu Trp Val Ala Trp Ile Ser Pro Tyr Gly Gly Ser Thr
        195                 200                 205

Tyr Tyr Ala Asp Ser Val Lys Gly Arg Phe Thr Ile Ser Ala Asp Thr
    210                 215                 220

Ser Lys Asn Thr Ala Tyr Leu Gln Met Asn Ser Leu Arg Ala Glu Asp
225                 230                 235                 240

Thr Ala Val Tyr Tyr Cys Ala Arg Arg His Trp Pro Gly Gly Phe Asp
                245                 250                 255

Tyr Trp Gly Gln Gly Thr Leu Val Thr Val Ser Ala Ala Ser Thr Lys
            260                 265                 270

Gly Pro Ser Val Phe Pro Leu Ala Pro Ser Ser Lys Ser Thr Ser Gly

275                 280                 285

Gly Thr Ala Ala Leu Gly Cys Leu Val Lys Asp Tyr Phe Pro Glu Pro
    290                 295                 300

Val Thr Val Ser Trp Asn Ser Gly Ala Leu Thr Ser Gly Val His Thr
305                 310                 315                 320

Phe Pro Ala Val Leu Gln Ser Ser Gly Leu Tyr Ser Leu Ser Ser Val
                325                 330                 335

Val Thr Val Pro Ser Ser Ser Leu Gly Thr Gln Thr Tyr Ile Cys Asn
            340                 345                 350

Val Asn His Lys Pro Ser Asn Thr Lys Val Asp Lys Lys Val Glu Pro
        355                 360                 365

Lys Ser Cys Asp Lys Thr His Thr Cys Pro Pro Cys Pro Ala Pro Glu
    370                 375                 380

Leu Leu Gly Gly Pro Ser Val Phe Leu Phe Pro Pro Lys Pro Lys Asp
385                 390                 395                 400

Thr Leu Met Asn Ser Arg Thr Pro Glu Val Thr Cys Val Val Val Asp
                405                 410                 415

Val Ser His Glu Asp Pro Glu Val Lys Phe Asn Trp Tyr Val Asp Gly
            420                 425                 430

Val Glu Val His Asn Ala Lys Thr Lys Pro Arg Glu Glu Gln Tyr Ala
        435                 440                 445

Ser Thr Tyr Arg Val Val Ser Val Leu Thr Val Leu His Gln Asp Trp
    450                 455                 460

Leu Asn Gly Lys Glu Tyr Lys Cys Lys Val Ser Asn Lys Ala Leu Pro
465                 470                 475                 480

Ala Pro Ile Glu Lys Thr Ile Ser Lys Ala Lys Gly Gln Pro Arg Glu
                485                 490                 495

Pro Gln Val Tyr Thr Leu Pro Pro Ser Arg Glu Glu Met Thr Lys Asn
            500                 505                 510

Gln Val Ser Leu Trp Cys Leu Val Lys Gly Phe Tyr Pro Ser Asp Ile
        515                 520                 525

Ala Val Glu Trp Glu Ser Asn Gly Gln Pro Glu Asn Asn Tyr Lys Thr
    530                 535                 540

Thr Pro Pro Val Leu Asp Ser Asp Gly Ser Phe Phe Leu Tyr Ser Lys
545                 550                 555                 560

Leu Thr Val Asp Lys Ser Arg Trp Gln Gln Gly Asn Val Phe Ser Cys
                565                 570                 575

Ser Val Met His Glu Ala Leu His Asn Gln Tyr Thr Gln Lys Ser Leu
            580                 585                 590

Ser Leu Ser Pro Gly Lys
        595

<210>  23
<211>  467
<212>  PRT
<213>  Artificial Sequence

<220>
<223>  fusion protein

<400>  23

Met Glu Phe Gly Leu Ser Trp Val Phe Leu Val Ala Leu Leu Arg Gly
1               5                   10                  15

Val Gln Cys Glu Val Gln Leu Val Glu Ser Gly Gly Gly Leu Val Gln
            20                  25                  30

Pro Gly Gly Ser Leu Arg Leu Ser Cys Ala Ala Ser Gly Phe Thr Phe
        35                  40                  45

Ser Asp Ser Trp Ile His Trp Val Arg Gln Ala Pro Gly Lys Gly Leu
    50                  55                  60

Glu Trp Val Ala Trp Ile Ser Pro Tyr Gly Gly Ser Thr Tyr Tyr Ala
65                  70                  75                  80

Asp Ser Val Lys Gly Arg Phe Thr Ile Ser Ala Asp Thr Ser Lys Asn
                85                  90                  95

Thr Ala Tyr Leu Gln Met Asn Ser Leu Arg Ala Glu Asp Thr Ala Val
            100                 105                 110

Tyr Tyr Cys Ala Arg Arg His Trp Pro Gly Gly Phe Asp Tyr Trp Gly
        115                 120                 125

Gln Gly Thr Leu Val Thr Val Ser Ser Ala Ser Thr Lys Gly Pro Ser

130                 135                 140

Val Phe Pro Leu Ala Pro Ser Ser Lys Ser Thr Ser Gly Gly Thr Ala
145                 150                 155                 160

Ala Leu Gly Cys Leu Val Lys Asp Tyr Phe Pro Glu Pro Val Thr Val
                165                 170                 175

Ser Trp Asn Ser Gly Ala Leu Thr Ser Gly Val His Thr Phe Pro Ala
            180                 185                 190

Val Leu Gln Ser Ser Gly Leu Tyr Ser Leu Ser Ser Val Val Thr Val
        195                 200                 205

Pro Ser Ser Ser Leu Gly Thr Gln Thr Tyr Ile Cys Asn Val Asn His
    210                 215                 220

Lys Pro Ser Asn Thr Lys Val Asp Lys Lys Val Glu Pro Lys Ser Cys
225                 230                 235                 240

Asp Lys Thr His Thr Cys Pro Pro Cys Pro Ala Pro Glu Leu Leu Gly
                245                 250                 255

Gly Pro Ser Val Phe Leu Phe Pro Pro Lys Pro Lys Asp Thr Leu Met
            260                 265                 270

Asn Ser Arg Thr Pro Glu Val Thr Cys Val Val Val Asp Val Ser His
        275                 280                 285

Glu Asp Pro Glu Val Lys Phe Asn Trp Tyr Val Asp Gly Val Glu Val
    290                 295                 300

His Asn Ala Lys Thr Lys Pro Arg Glu Glu Gln Tyr Ala Ser Thr Tyr
305                 310                 315                 320

Arg Val Val Ser Val Leu Thr Val Leu His Gln Asp Trp Leu Asn Gly
                325                 330                 335

Lys Glu Tyr Lys Cys Lys Val Ser Asn Lys Ala Leu Pro Ala Pro Ile
            340                 345                 350

Glu Lys Thr Ile Ser Lys Ala Lys Gly Gln Pro Arg Glu Pro Gln Val
        355                 360                 365

Tyr Thr Leu Pro Pro Ser Arg Glu Glu Met Thr Lys Asn Gln Val Ser
    370                 375                 380

Leu Trp Cys Leu Val Lys Gly Phe Tyr Pro Ser Asp Ile Ala Val Glu
385                 390                 395                 400

Trp Glu Ser Asn Gly Gln Pro Glu Asn Asn Tyr Lys Thr Thr Pro Pro
                405                 410                 415

Val Leu Asp Ser Asp Gly Ser Phe Phe Leu Tyr Ser Lys Leu Thr Val
            420                 425                 430

Asp Lys Ser Arg Trp Gln Gln Gly Asn Val Phe Ser Cys Ser Val Met
        435                 440                 445

His Glu Ala Leu His Asn Gln Tyr Thr Gln Lys Ser Leu Ser Leu Ser
    450                 455                 460

Pro Gly Lys
465

<210>  24
<211>  236
<212>  PRT
<213>  Artificial Sequence

<220>
<223>  fusion protein

<400>  24

Met Lys Tyr Leu Leu Pro Thr Ala Ala Ala Gly Leu Leu Leu Leu Ala
1               5                   10                  15

Ala Gln Pro Ala Met Ala Asp Ile Gln Met Thr Gln Ser Pro Ser Ser
            20                  25                  30

Leu Ser Ala Ser Val Gly Asp Arg Val Thr Ile Thr Cys Arg Ala Ser
        35                  40                  45

Gln Asp Val Ser Thr Ala Val Ala Trp Tyr Gln Gln Lys Pro Gly Lys
    50                  55                  60

Ala Pro Lys Leu Leu Ile Tyr Ser Ala Ser Phe Leu Tyr Ser Gly Val
65                  70                  75                  80

Pro Ser Arg Phe Ser Gly Ser Gly Ser Gly Thr Asp Phe Thr Leu Thr
                85                  90                  95

Ile Ser Ser Leu Gln Pro Glu Asp Phe Ala Thr Tyr Tyr Cys Gln Gln
            100                 105                 110

Tyr Leu Tyr His Pro Ala Thr Phe Gly Gln Gly Thr Lys Val Glu Ile

115                 120                 125

Lys Arg Thr Val Ala Ala Pro Ser Val Phe Ile Phe Pro Pro Ser Asp
    130                 135                 140

Glu Gln Leu Lys Ser Gly Thr Ala Ser Val Val Cys Leu Leu Asn Asn
145                 150                 155                 160

Phe Tyr Pro Arg Glu Ala Lys Val Gln Trp Lys Val Asp Asn Ala Leu
                165                 170                 175

Gln Ser Gly Asn Ser Gln Glu Ser Val Thr Glu Gln Asp Ser Lys Asp
            180                 185                 190

Ser Thr Tyr Ser Leu Ser Ser Thr Leu Thr Leu Ser Lys Ala Asp Tyr
        195                 200                 205

Glu Lys His Lys Val Tyr Ala Cys Glu Val Thr His Gln Gly Leu Ser
    210                 215                 220

Ser Pro Val Thr Lys Ser Phe Asn Arg Gly Glu Cys
225                 230                 235